# EUROPEAN PATENT SPECIFICATION

(11) **EP 0 874 863 B1**
(45) Date of publication and mention of the grant of the patent: **29.01.2003**
(21) Application number: 96940766.7
(22) Date of filing: 06.11.1996
(51) Int. Cl.: C07H 17/08, A61K 31/70

(54) **3-DEOXY-3-DESCLADINOSE DERIVATIVES OF ERYTHROMYCINS A AND B**
3-DEOXY-3-DESCLADINOSEDERIVATE VON ERYTHROMYCIN A UND B
DERIVES 3-DESOXY-3-DESCLADINOSE DES ERYTHROMYCINES A ET B

(30) Priority: 08.11.1995 US 6342 P; 10.10.1996 US 729269
(43) Date of publication of application: 04.11.1998
(73) Proprietor: ABBOTT LABORATORIES, Abbott Park, Illinois 60064-3500 (US)
(72) Inventor: OR, Yat, Sun, Libertyville, IL 60048 (US); CHU, Daniel, T., Santa Clara, CA 95051 (US); ELLIOTT, Richard, L., Grayslake, IL 60030 (US); PIREH, Daisy, Lincolnshire, IL 60069 (US)
(74) Representative: Modiano, Guido, Dr.-Ing.
(86) International application number: US9617892
(87) International publication number: WO97017357

(56) References cited:
- EP-A- 0 487 411
- EP-A- 0 619 319
- J. MED. CHEM., vol. 17, 1974, pages 953-6, XP002024503 R.A. LEMAHIEU ET AL.: "Glycoside Cleavage Reactions on Erythromycin A"

## Description

### Technical Field

The present invention relates to novel semisynthetic macrolides having antibacterial activity and useful in the treatment and prevention of bacterial infections. More particularly, the invention relates to 3-deoxy-3-descladinose-derivatives of erythromycin A and B, compositions containing such compounds and methods for using the same.

### Background Of The Invention

Erythromycins A through D, represented by formula (E), 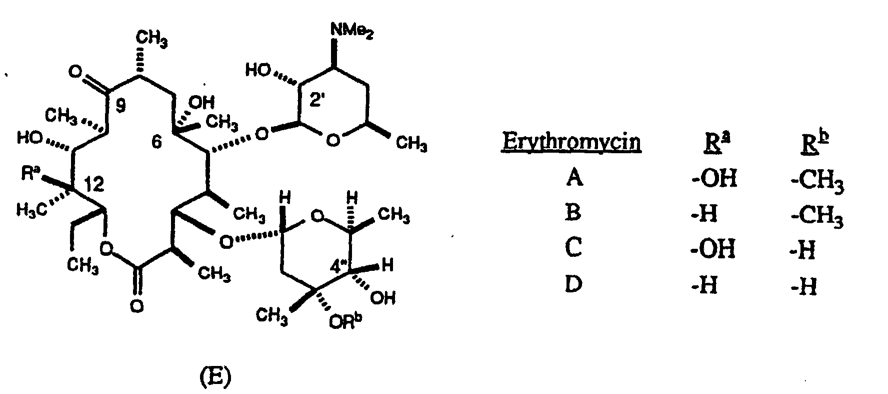 are well-known and potent antibacterial agents, used widely to treat and prevent bacterial infection. As with other antibacterials, however, bacterial strains having resistance or insufficient susceptibility to erythromycin have been identified. Therefore, there is a continuing need to identify new antibiotic compounds to which resistance has not developed. Also, new antibiotics having less potential to developing resistance are desired. Consequently, numerous investigators have prepared chemical derivatives of erythromycin in an attempt to obtain analogs having modified or improved profiles of antibiotic activity.

Erythromycin analogs having modifications at the C-3 or C-6 position have been disclosed in United States Patent No. 5,403,923, issued April 4, 1995, European application EP 487411, published May 27, 1992, French Patent Number 2,697,524, issued May 6, 1994, European application EP 596802, published May 11, 1994, PCT application WO 9321200, published October 28, 1993, United States Patent No. 4,331,803, issued May 25, 1982, and United States Patent No. 5,141,926, issued August 25, 1992.

### Summary Of The Invention

The present invention provides a novel class of 3-deoxy-3-descladinose derivatives of erythromycins A and B which possess antibacterial activity.

In one aspect of the present invention are disclosed novel derivatives of erythromycin, as well as the pharmaceutically acceptable salts and esters thereof, having a formula selected from the group: 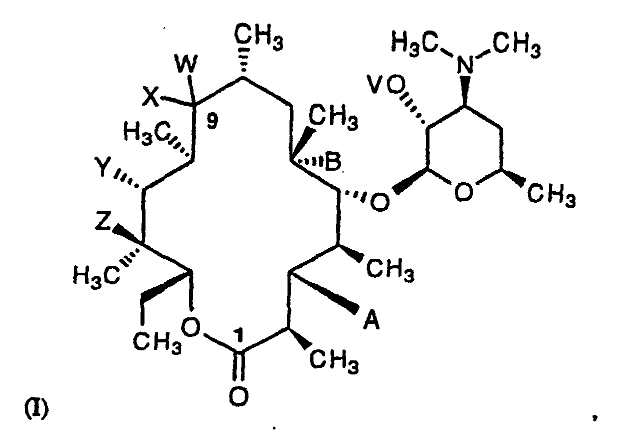 wherein:
**A** is hydrogen,
**B** is selected from the group consisting of hydrogen, hydroxy, and methoxy;
**V** is hydrogen or a hydroxy-protecting group;
**W** is hydrogen; and **X** is selected from the group consisting of:
   (a) NR²R³, wherein R² and R³ are independently selected from the group consisting of hydrogen, C₁-C₆-alkyl, C₁-C₆-alkylamino, aryl-C₁-C₆-alkyl, or heteroaryl-C₁-C₆-alkyl, or wherein R² and R³ are taken together with the nitrogen atom to which they are attached to form a 5-to-7-membered saturated ring; and
   (b) NR₄-(C=O)-R¹, wherein R¹ is as described above and R⁴ is hydrogen or C₁-C₆-alkyl; or
**W** and **X** are taken together and represent =O, =N-O-R², wherein R² is as described above, or =N-R⁵, wherein R⁵ is selected from the group consisting of:
   (a) hydrogen,
   (b) C₁-C₆-alkyl,
   (c) substituted C₁-C₆-alkyl,
   (d) -(CH₂)ₘ-L-(CH₂)ₙ-M-CH₂-H, wherein m=1-6, n=1-6, L and M are both oxygen, L is oxygen and M is absent, or M is oxygen and L is absent,
   (e) -(CH₂)ₘ-L-aryl-M-(CH₂)ₙ-H, wherein m=1-6, n=1-6, L and M are both oxygen, L is oxygen and M is absent, or M is oxygen and L is absent, and
   (f) -(CH₂)ₘL-(CH₂)ₙ-M-aryl-H, wherein m=1-6, n=1-6, L and M are both oxygen, L is oxygen and M is absent, or M is oxygen and L is absent;
**Y** is -OH, or -OR⁶ wherein R⁶ is a hydroxy-protecting group; and
**Z** is hydrogen, -OH, or -OR⁷ wherein R⁷ is a hydroxy-protecting group; 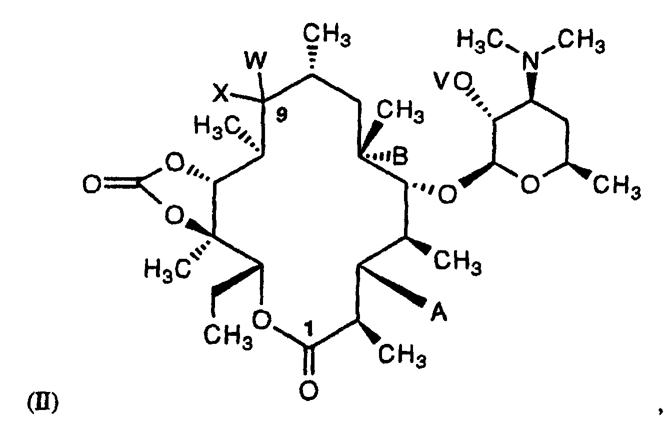
wherein **A, B, V, W,** and **X** are as defined above, or 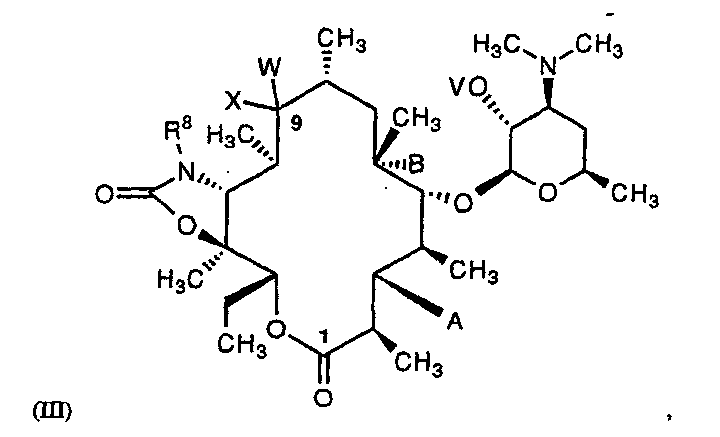 wherein **A, B, V, W,** and **X** are as defined above, and R⁸ is selected from the group consisting of:
(a) hydrogen,
(b) C₁-C₆-alkyl,
(c) aryl,
(d) aryl-C₁-C₆-alkyl,
(e) heteroaryl-C₁-C₆-alkyl,
(f) cycloalkyl,
(g) cycloalkyl-C₁-C₆-alkyl,
(h) -NH-R⁹, wherein R⁹ is selected from the group consisting of:
   (aa) C₁-C₆-alkyl,
   (bb) aryl-C₁-C₆-alkyl,
   (cc) heteroaryl-C₁-C₆-alkyl,
   (dd) C₃-C₇-cycloalkyl,
   (ee) C₃-C₇-cycloalkyl-C₁-C₆-alkyl,
   (ff) -CO-C₁-C₆-alkyl,
   (gg) -CO-aryl,
   (hh) -CO-C₁-C₆-alkyl-aryl-; and
(i) -N=CH-R¹⁰, wherein R¹⁰ is selected from the group consisting of:
   (aa) C₁-C₆-alkyl,
   (bb) aryl,
   (cc) aryl-C₁-C₆-alkyl,
   (dd) heteroaryl-C₁-C₆-alkyl,
   (ee) C₃-C₇-cycloalkyl, and
   (ff) C₃-C₇-cycloalkyl-C₁-C₆-alkyl.

In a second aspect of the present invention are disclosed pharmaceutical compositions comprising a therapeutically effective amount of the above compounds in combination with a pharmaceutically acceptable carrier.

The pharmaceutical compositions can be used for treating or preventing bacterial infections in a patient in need of such treatment or prevention, comprising administering to the patient a therapeutically effective amount of one of the above compounds.

Also one aspect of the invention includes processes for the preparation of the compounds of the invention.

### Detailed Description Of The Invention

The term "C₁-C₆-alkyl", as used herein, refers to saturated, straight- or branched-chain hydrocarbon radicals containing from one to six carbon atoms including, but not limited to, methyl, ethyl, propyl, isopropyl, *n*-butyl, *tert*-butyl, neopentyl and n-hexyl, or to a mono-unsaturated straight- or branched-chain hydrocarbon radicals containing from three to six carbon atoms including, but not limited to, propenyl, *n*-butenyl, and n-hexenyl.

The term "C₁-C₆-alkylamino", as used herein, refers to an amino radical having appended thereto one or two C₁-C₆-alkyl radicals as defined above, such as for example methylamino, dimethyl amino, ethylamino, diethylamino and butylamino.

The term "C₁-C₆-alkylamino-C₁-C₆-alkyl", as used herein, refers to a C₁-C₆-alkyl radical having appended thereto, by replacement of a hydrogen atom, a C₁-C₆-alkylamino radical, as defined above.

The term "aryl", as used herein, refers to an unsubstituted carbocyclic aromatic radical, including, for example, phenyl and 1- or 2-naphthyl.

The term "aprotic solvent" as used herein refers to a solvent that is relatively inert to proton activity, *i.e.,* not acting as a proton-donor. Examples include, but are not limited to, hydrocarbons, such as hexane and toluene, for example, halogenated hydrocarbons, such as, for example, methylene chloride, ethylene chloride, chloroform, and the like, heterocyclic compounds, such as, for example, tetrahydrofuran and N-methylpyrrolidinone, and ethers such as diethyl ether, bis-methoxymethyl ether. Such compounds are well known to those skilled in the art, and it will be obvious to those skilled in the art that individual solvents or mixtures thereof may be preferred for specific compounds and reaction conditions, depending upon such factors as the solubility of reagents, reactivity of reagents and preferred temperature ranges, for example. Further discussions of aprotic solvents may be found in organic chemistry textbooks or in specialized monographs, for example: Organic Solvents Physical Properties and Methods of Purification, 4th edition, edited by John A. Riddick, *et al*., Vol. II, in the Techniques of Chemistry, Series, John Wiley & Sons, NY, 1986.

The term "aryl-C₁-C₆-alkyl", as used herein, refers to a C₁-C₆-alkyl radical having appended thereto, by replacement of a hydrogen atom, an aryl radical, as defined above.

The term "heteroaryl", as used herein, refers to a cyclic aromatic radical having from five to ten ring atoms of which one ring atom is selected from S, O and N; zero, one or two ring atoms are additional heteroatoms independently selected from S, O and N; and the remaining ring atoms are carbon, the radical being joined to the rest of the molecule via any of the ring atoms, such as, for example, pyridinyl, pyrazinyl, pyrimidinyl, pyrrolyl, pyrazolyl, imidazolyl, thiazolyl, oxazolyl, isooxazolyl, thiadiazolyl, oxadiazolyl, thiophenyl, furanyl, quinolinyl, isoquinolinyl, and the like.

The term "heteroaryl-C₁-C₆-alkyl", as used herein, refers to a C₁-C₆-alkyl radical having appended thereto, by replacement of a hydrogen atom, a heteroaryl radical as defined above.

The term "hydroxy-protecting group", as used herein, refers to an easily removable group to which are known in the art to protect a hydroxyl group against undesirable reaction during synthetic procedures and to be selectively removable. The use of hydroxy-protecting groups is well known in the art for protecting groups against undesirable reactions during a synthetic procedure and many such protecting groups are known, cf, for example, T. H. Greene and P. G. M. Wuts, Protective Groups in Organic Synthesis, 2nd edition, John Wiley & Sons, New York (1991). Examples of hydroxy-protecting groups include, but are not limited to, methylthiomethyl, *tert*-dimethylsilyl, *tert*-butyldiphenylsilyl, acyl substituted with an aromatic group and the like.

The term "protected-hydroxy" refers to a hydroxy group protected with a hydroxy protecting group, as defined above, including benzoyl, acetyl, trimethylsilyl, triethylsilyl, methoxymethyl groups, for example.

The term "substituted C₁-C₆-alkyl", as used herein, refers to a C₁-C₆-alkyl radical as defined above wherein one, two or three of the hydrogen atoms thereon have been independently replaced with a Cl, Br, F, OH, methoxy, ethoxy, propoxy, amino, C₁-C₆-alkylamino, as defined below, or C₁-C₆-alkylamino-C₁-C₆-alkyl, as defined below, group.

The term "substituted-aryl", as used herein, refers to an aryl radical having appended thereto by independent replacement of one, two or three of the hydrogen atoms thereon with Cl, Br, F, OH, methoxy, ethoxy, propoxy, C₁-C₆-alkyl, trifluoromethyl or methoxymethyl groups.

The term "substituted aryl-C₁-C₆-alkyl", as used herein, refers to refers to a C₁-C₆-alkyl radical having appended thereto, by replacement of a hydrogen atom, an substituted aryl radical, as defined above.

In one embodiment of the invention are compounds of Formula (I) above, wherein **A, B, V, W, X, Y** and **Z** are as defined above.

In a second embodiment of the invention are compounds of Formula (II) above, wherein **A, B, V, W,** and **X** are as defined above.

In another embodiment of the invention are compounds of Formula (III) above, wherein **A, B, V, W, X** and **R**^{**8**} are as defined above.

In a preferred embodiment of the invention are compounds of Formula (III) above, wherein **A, B, V, W, X** and **R**^{**8**} are as defined above, and R⁸ is -NH-R⁹, wherein R⁹ is selected from the group consisting of:
(aa) C₁-C₆-alkyl,
(bb) aryl-C₁-C₆-alkyl,
(cc) heteroaryl-C₁-C₆-alkyl,
(dd) C₃-C₇-cycloalkyl,
(ee) C₃-C₇-cycloalkyl-C₁-C₆-alkyl,
(ff) -CO-C₁-C₆-alkyl,
(gg) -CO-aryl,and
(hh) -CO-C₁-C₆-alkyl-aryl-.

In another embodiment of the invention are compounds of Formula (I) above wherein **A** is hydrogen and **Z** is -OH or -O-R⁷.

Representative compounds of the invention include:
Compound of Formula (I) wherein: A=H; B=methoxy; V=H; W and X taken together are =O; Y=Z= -OH;
Compound of Formula (I) wherein: A=H; B=methoxy; V=H; W and X taken together are =N-OH; Y=Z= -OH;
Compound of Formula (II) wherein: A=H; B=methoxy; V=H; W and X taken together are =O;
Compound of Formula (III) wherein: A=H; B=methoxy; V=H; W and X taken together are =O; R⁸ = -CH₂-CH₂-CH₂-CH₂-phenyl;
Compound of Formula (III) wherein: A=H; B=methoxy; V=H; W and X taken together are =O; R⁸ = -CH₂-CH₂-phenyl-4-O-phenyl;
Compound of Formula (I) wherein: A=H; B=methoxy; V=H; W and X taken together are =N-O-CH₃; Y=Z= -OH;
Compound of Formula (I) wherein: A=H; B=methoxy; V=H; W and X taken together are =N-O-CH₂-CH₃; Y=Z= -OH; and
Compound of Formula (I) wherein: A=H; B=methoxy; V=H; W and X taken together are =N-O-CH₂-O-CH₂-CH₂-O-CH₃; Y=Z= -OH;
Compound of Formula (III) wherein: A=H; B=methoxy; V=H; W and X taken together are =O; R⁸ = NH₂;
Compound of Formula (I) wherein: A=H; B=hydroxy; V=H; W and X taken together are =O; Y=Z= -OH;
Compound of Formula (III) wherein: A=H; B=methoxy; V=H; W and X taken together are =O; R⁸ = -NH-CO-CH₃;
Compound of Formula (III) wherein: A=H; B=methoxy; V=H; W and X taken together are =O; R⁸ = -N=CH-phenyl;
Compound of Formula (III) wherein: A=H; B=methoxy; V=H; W and X taken together are =O; R⁸ = -NH₂-CH₂-phenyl;
Compound of Formula (III) wherein: A=H; B=methoxy; V=H; W and X taken together are =O; R⁸ = -CH₂-CH₂-CH₂-phenyl;
Compound of Formula (III) wherein: A=H; B=methoxy; V=H; W and X taken together are O; R⁸ = NH-CO-C₆H₅;
Compound of Formula (III) wherein: A=H; B=methoxy; V=H; W and X taken together are O; R⁸ = NH-CO-CH₂-C₆H₅;
Compound of Formula (III) wherein: A=H; B=methoxy; V=H; W and X taken together are O; R⁸ = NH-CO-CH₂CH₂C₆H₅;
Compound of Formula (III) wherein: A=H; B=methoxy; V=H; W and X taken together are O; R⁸ = NH-CO-CH₂-CH₂-C₆H₅;
Compound of Formula (III) wherein: A=H; B=methoxy; V=H; W and X taken together are O; R⁸ = NH-CO-CH₂-COOMe;
Compound of Formula (III) wherein: A=H; B=methoxy; V=H; W and X taken together are O; R⁸ = NH-CO-CH₂-CH₂-(4-quinoyl);
Compound of Formula (III) wherein: A=H; B=methoxy; V=H; W and X taken together are O; R⁸ = N=CH-CH3;
Compound of Formula (III) wherein: A=H; B=methoxy; V=H; W and X taken together are O; R⁸ = N=CH-CH₂C₆H₅;
Compound of Formula (III) wherein: A=H; B=methoxy; V=H; W and X taken together are O; R⁸ = N=CH-CH₂CH₂C₆H₅;
Compound of Formula (III) wherein: A=H; B=methoxy; V=H; W and X taken together are O; R⁸ = N=CH-CH=CH-phenyl;
Compound of Formula (III) wherein: A=H; B=methoxy; V=H; W and X taken together are O; R⁸ = N=CH-CH₂-CH₂-(4-quinoyl);
Compound of Formula (III) wherein: A=H; B=methoxy; V=H; W and X taken together are O; R⁸ = N=CH-cyclopentane;
Compound of Formula (III) wherein: A=H; B=methoxy; V=H; W and X taken together are O; R⁸ = N=CH-CH₂-cyclohexane;
Compound of Formula (III) wherein: A=H; B=methoxy; V=H; W and X taken together are O; R⁸ = NH-ethyl;
Compound of Formula (III) wherein: A=H; B=methoxy; V=H; W and X taken together are O; R⁸ = NH-CH₂C₆H₅;
Compound of Formula (III) wherein: A=H; B=methoxy; V=H; W and X taken together are O; R⁸ = NH-;
Compound of Formula (III) wherein: A=H; B=methoxy; V=H; W and X taken together are O; R⁸ = NH-CH₂-CH₂-CH₂-C₆H₅;
Compound of Formula (III) wherein: A=H; B=methoxy; V=H; W and X taken together are O; R⁸ = NH-CH₂-CH=CH-phenyl;
Compound of Formula (III) wherein: A=H; B=methoxy; V=H; W and X taken together are O; R⁸ = NH-CH₂-CH₂-CH₂-(4-quinoyl);
Compound of Formula (III) wherein: A=H; B=methoxy; V=H; W and X taken together are O; R⁸ = NH-CH-CH₂-cyclohexane;
Compound of Formula (III) wherein: A=H; B=methoxy; V=H; W and X taken together are O; R⁸ = H;
Compound of Formula (III) wherein: A=H; B=methoxy; V=H; W and X taken together are O; R⁸ = Me;
Compound of Formula (III) wherein: A=H; B=methoxy; V=H; W and X taken together are O; R⁸ = benzyl;
Compound of Formula (III) wherein: A=H; B=methoxy; V=H; W and X taken together are O; R⁸ = phenylethyl;
Compound of Formula (III) wherein: A=H; B=methoxy; V=H; W and X taken together are O; R⁸ = phenylpropyl;
Compound of Formula (III) wherein: A=H; B=methoxy; V=H; W and X taken together are O; R⁸ = -CH₂-CH₂-CH₂-CH₂-(4-quinoyl);
Compound of Formula (III) wherein: A=H; B=methoxy; V=H; W and X taken together are O; R⁸ = cyclohexyl;
Compound of Formula (III) wherein: A=H; B=methoxy; V=H; W and X taken together are O; R⁸ = -CH₂--CH₂-cyclohexyl; and
Compound of Formula ((III) wherein) wherein: A=H; B=methoxy; V=H; W and X taken together are =O; R⁸ = phenyl.

Selected representative compounds of the invention include:
Compound of Formula (I) wherein: A=H; B=methoxy; V=H; W and X taken together are =O; Y=Z= -OH;
Compound of Formula (I) wherein: A=H; B=methoxy; V=H; W and X taken together are =N-OH; Y=Z= -OH;
Compound of Formula (II) wherein: A=H; B=methoxy; V=H; W and X taken together are =O;
Compound of Formula (III) wherein: A=H; B=methoxy; V=H; W and X taken together are =O; R⁸ = -CH₂-CH₂-CH₂-CH₂-phenyl;
Compound of Formula (III) wherein: A=H; B=methoxy; V=H; W and X taken together are =O; R⁸ = -CH₂-CH₂-phenyl-4-O-phenyl;
Compound of Formula (I) wherein: A=H; B=methoxy; V=H; W and X taken together are =N-O-CH₃; Y=Z= -OH;
Compound of Formula (I) wherein: A=H; B=methoxy; V=H; W and X taken together are =N-O-CH₂-CH₃; Y=Z= -OH; and
Compound of Formula (I) wherein: A=H; B=methoxy; V=H; W and X taken together are =N-O-CH₂-O-CH₂-CH₂-O-CH₃; Y=Z= -OH.

One object of the present invention is to provide a process for the preparation of 3-deoxy-3-descladinose derivatives of erythromycins A and B having the formula: 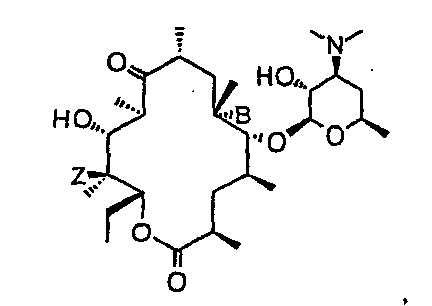 wherein **B** is hydrogen, hydroxy or methoxy, and **Z** is H or OH, the method comprising:
(a) reacting a compound having the formula: 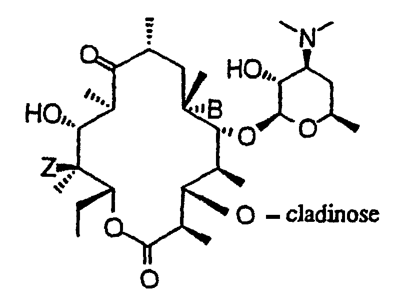 wherein **B,** and **Z** are as defined above, with a strong acid, preferably 0.5-1.5 N HCl or dichloroacetic acid, in a suspension in aqueous alcohol, preferably, methanol, ethanol, or isopropanol, by stirring at ambient temperature for 0.5 to 24 hours to give a first intermediate compound having the formula: 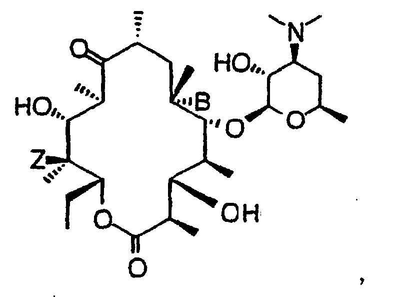 wherein **B**, and **Z** are as defined above;
(b) reacting the first intermediate compound with an excess of NaH at from 0 to -30°C under an inert atmosphere, in an aprotic solvent such as DMF, THF, or Et₂O, for example, followed by reaction of the intermediate anion with CS₂ and CH₃I at -5 to 10°C, to form a second intermediate 3-O-xanthyl compound having the formula: 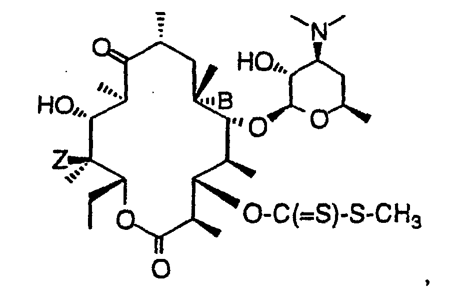 wherein **B,** and **Z** are as defined above;
(c) reacting the second intermediate compound with 1.1-1.3 equivalents of Bu₃SnH under an inert atmosphere in the presence of a catalytic amount of AIBN or other suitable radical initiator, in a solvent suitable for a free radical reaction, such as benzene or toluene, for example, at reflux conditions, and in the case wherein B = OH and M = NOR², followed by deoximation with, for example, NaNO₂ in the presence of aq. acid/MeOH, or using NaHSO₃/formic acid in H₂O/isopropanol at r.t to reflux to afford the desired compound.

As used herein, the term "pharmaceutically acceptable salt" refers to those salts which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts are well known in the art. For example, S. M. Berge, *et al*.. describe pharmaceutically acceptable salts in detail in *J. Pharmaceutical Sciences*, 66: 1-19 (1977), incorporated herein by reference. The salts can be prepared *in situ* during the final isolation and purification of the compounds of the invention, or separately by reacting the free base function with a suitable organic acid. Examples of pharmaceutically acceptable, nontoxic acid addition salts are salts of an amino group formed, with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, *p*-toluenesulfonate, undecanoate, valerate salts, and the like. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, loweralkyl sulfonate and aryl sulfonate.

As used herein, the term "pharmaceutically acceptable ester" refers to esters which hydrolyze *in vivo* and include those that break down readily in the human body to leave the parent compound or a salt thereof. Suitable ester groups include, for example, those derived from pharmaceutically acceptable aliphatic carboxylic acids, particularly alkanoic, alkenoic, cycloalkanoic and alkanedioic acids, in which each alkyl or alkenyl moiety advantageously has not more than six carbon atoms. Examples of particular esters includes formates, acetates, propionates, butyates, acrylates and ethylsuccinates.

The pharmaceutical compositions of the present invention comprise a therapeutically effective amount of a compound of the present invention formulated together with one or more pharmaceutically acceptable carriers. As used herein, the term "pharmaceutically acceptable carrier" means a non-toxic, inert solid, semi-solid or liquid filler, diluent, encapsulating material or formulation auxiliary of any type. Some examples of materials which can serve as pharmaceutically acceptable carriers are sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil; safflower oil; sesame oil; olive oil; corn oil and soybean oil; glycols; such a propylene glycol; esters such as ethyl oleate and ethyl laurate; agar; buffering agents such as magnesium hydroxide and aluminum hydroxide; alginic acid; pyrogen-free water; isotonic saline; Ringer's solution; ethyl alcohol, and phosphate buffer solutions, as well as other non-toxic compatible lubricants such as sodium lauryl sulfate and magnesium stearate, as well as coloring agents, releasing agents, coating agents, sweetening, flavoring and perfuming agents, preservatives and antioxidants can also be present in the composition, according to the judgment of the formulator. The pharmaceutical compositions of this invention can be administered to humans and other animals orally, rectally, parenterally, intracisternally, intravaginally, intraperitoneally, topically (as by powders, ointments, or drops), bucally, or as an oral or nasal spray.

Liquid dosage forms for oral administration include pharmaceutically acceptable emulsions, microemulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, and perfuming agents.

Injectable preparations, for example, sterile injectable aqueous or oleaginous suspensions may be formulated according to the known art using suitable dispersing or wetting agents and suspending agents. The sterile injectable preparation may also be a sterile injectable solution, suspension or emulsion in a nontoxic parenterally acceptable diluent or solvent, for example, as a solution in 1,3-butanediol. Among the acceptable vehicles and solvents that may be employed are water, Ringer's solution, U.S.P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectables.

The injectable formulations can be sterilized, for example, by filtration through a bacterial-retaining filter, or by incorporating sterilizing agents in the form of sterile solid compositions which can be dissolved or dispersed in sterile water or other sterile injectable medium prior to use.

In order to prolong the effect of a drug, it is often desirable to slow the absorption of the drug from subcutaneous or intramuscular injection. This may be accomplished by the use of a liquid suspension of crystalline or amorphous material with poor water solubility. The rate of absorption of the drug then depends upon its rate of dissolution which, in turn, may depend upon crystal size and crystalline form. Alternatively, delayed absorption of a parenterally administered drug form is accomplished by dissolving or suspending the drug in an oil vehicle. Injectable depot forms are made by forming microencapsule matrices of the drug in biodegradable polymers such as polylactide-polyglycolide. Depending upon the ratio of drug to polymer and the nature of the particular polymer employed, the rate of drug release can be controlled. Examples of other biodegradable polymers include poly(orthoesters) and poly(anhydrides) Depot injectable formulations are also prepared by entrapping the drug in liposomes or microemulsions which are compatible with body tissues.

Compositions for rectal or vaginal administration are preferably suppositories which can be prepared by mixing the compounds of this invention with suitable non-irritating excipients or carriers such as cocoa butter, polyethylene glycol or a suppository wax which are solid at ambient temperature but liquid at body temperature and therefore melt in the rectum or vaginal cavity and release the active compound.

Solid dosage forms for oral administration include capsules, tablets, pills, powders, and granules. In such solid dosage forms, the active compound is mixed with at least one inert, pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft and hard-filled gelatin capsules using such excipients as lactose or milk sugar as well as high molecular weight polyethylene glycols and the like.

The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferentially, in a certain part of the intestinal tract, optionally, in a delayed manner. Examples of embedding compositions which can be used include polymeric substances and waxes.

Solid compositions of a similar type may also be employed as fillers in soft and hard-filled gelatin capsules using such excipients as lactose or milk sugar as well as high molecular weight polethylene glycols and the like.

The active compounds can also be in micro-encapsulated form with one or more excipients as noted above. The solid dosage forms of tablets, dragees, capsules, pills, and granules can be prepared with coatings and shells such as enteric coatings, release controlling coatings and other coatings well known in the pharmaceutical formulating art. In such solid dosage forms the active compound may be admixed with at least one inert diluent such as sucrose, lactose or starch. Such dosage forms may also comprise, as is normal practice, additional substances other than inert diluents, *e*.*g*., tableting lubricants and other tableting aids such a magnesium stearate and microcrystalline cellulose. In the case of capsules, tablets and pills, the dosage forms may also comprise buffering agents. They may optionally contain opacifying agents and can also be of a composition that they release the active ingredient(s) only, or preferentially, in a certain part of the intestinal tract, optionally, in a delayed manner. Examples of embedding compositions which can be used include polymeric substances and waxes.

Dosage forms for topical or transdermal administration of a compound of this invention include ointments, pastes, creams, lotions, gels, powders, solutions, sprays, inhalants or patches. The active component is admixed under sterile conditions with a pharmaceutically acceptable carrier and any needed preservatives or buffers as may be required. Ophthalmic formulation, ear drops, eye ointments, powders and solutions are also contemplated as being within the scope of this invention.

The ointments, pastes, creams and gels may contain, in addition to an active compound of this invention, excipients such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to the compounds of this invention, excipients such as lactose, talc, silicic acid, aluminum hydroxide, calcium silicates and polyamide powder, or mixtures of these substances. Sprays can additionally contain customary propellants such as chlorofluorohydrocarbons.

Transdermal patches have the added advantage of providing controlled delivery of a compound to the body. Such dosage forms can be made by dissolving or dispensing the compound in the proper medium. Absorption enhancers can also be used to increase the flux of the compound across the skin. The rate can be controlled by either providing a rate controlling membrane or by dispersing the compound in a polymer matrix or gel.

According to the methods of treatment of the present invention, bacterial infections are treated or prevented in a patient such as a human or lower mammal by administering to the patient a therapeutically effective amount of a compound of the invention, in such amounts and for such time as is necessary to achieve the desired result. By a "therapeutically effective amount" of a compound of the invention is meant a sufficient amount of the compound to treat bacterial infections, at a reasonable benefit/risk ratio applicable to any medical treatment. It will be understood, however, that the total daily usage of the compounds and compositions of the present invention will be decided by the attending physician within the scope of sound medical judgment. The specific therapeutically effective dose level for any particular patient will depend upon a variety of factors including the disorder being treated and the severity of the disorder; the activity of the specific compound employed; the specific composition employed; the age, body weight, general health, sex and diet of the patient; the time of administration, route of administration, and rate of excretion of the specific compound employed; the duration of the treatment; drugs used in combination or coincidental with the specific compound employed; and like factors well known in the medical arts.

The total daily dose of the compounds of this invention administered to a human or other mammal in single or in divided doses can be in amounts, for example, from 0.01 to 50 mg/kg body weight or more usually from 0.1 to 25 mg/kg body weight. Single dose compositions may contain such amounts or submultiples thereof to make up the daily dose. In general, treatment regimens according to the present invention comprise administration to a patient in need of such treatment from about 10 mg to about 1000 mg of the compound(s) of this invention per day in single or multiple doses.

Numerous asymmetric centers may exist in the compounds of the present invention. Except where otherwise noted, the present invention contemplates the various stereoisomers and mixtures thereof. Accordingly, whenever a bond is represented by a wavy line, it is intended that a mixture of stereo-orientations or an individual isomer of assigned or unassigned orientation may be present.

### Abbreviations

Abbreviations which may have been used in the descriptions of the scheme and the examples that follow are: AIBN for azobisisobutyronitrile; Bu₃SnH for tributyltin hydride; CDI for carbonyldiimidazole; DBU for 1,8-diazabicyclo-[5.4.0]undec-7-ene; DEAD for diethylazodicarboxylate; DMF for dimethyl formamide; DPPA for diphenylphosphoryl azide; EtOAc for ethyl acetate; MeOH for methanol; NaN(TMS)₂ for sodium bis(trimethylsilyl)amide; NMMO for N-methyl-morpholine N-oxide; TEA for triethylamine; THF for tetrahydrofuran; TPP for triphenylphosphine.

### Synthetic Methods

The compounds and processes of the present invention will be better understood in connection with the following synthetic schemes which illustrate the methods by which the compounds of the invention may be prepared. The groups **A, B, V, W, X, Y** and **Z** are as defined above unless otherwise noted below.

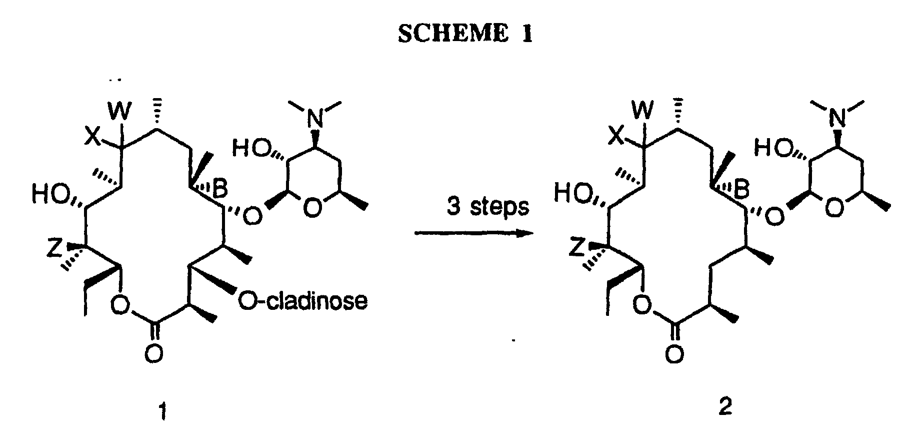

In accordance with Scheme 1 an erythromycin A (1, X+W = O, Z=OH, B = H or OMe) or erythromycin B (1, X+W = O, Z=H, B = H or OMe), or alternatively an oxime derivative of erythromycin A (1, X+W = NOR², as defined above, Z=OH, B = OH) or erythromycin B (1, X+W = NOR², Z=H, B = OH) compound (1) is converted into the desired 3-deoxy compound (2) by a series of reactions. The first removes the cladinose sugar moiety by reaction of the macrolide with a strong acid, for example 0.5-1.5 N HCl or dichloroacetic acid, in a suspension in aqueous alcohol, such as for example, methanol, ethanol, or isopropanol, by stirring at ambient temperature for 0.5 to 24 hours, for example. After basification of the reaction mixture with a base such as an alkali metal base, the precipitated product is washed and dried. The 3-O-descladinose compound is then dissolved in an aprotic solvent such as THF, then reacted with an excess of NaH at from 0 to -30°C under an inert atmosphere, followed by reaction of the intermediate anion with CS₂ and CH₃I at -5 to 10°C, to form a 3-O-xanthyl compound. This xanthate intermediate is then reacted with 1.1-1.3 equivalents of Bu₃SnH under an inert atmosphere in the presence of a catalytic amount of AIBN or other suitable radical initiator, in a solvent suitable for a free radical reaction, such as benzene or toluene, for example, at reflux conditions to afford the desired compound (2). In the case for (2) where B = OH and X+W = NOR, the 9-keto analog (X+W = O) can be prepared via known deoximation methods using, for example, NaNO₂ in the presence of aq. acid/MeOH, or using NaHSO₃/formic acid in H₂O/isopropanol at r.t to reflux, for example.

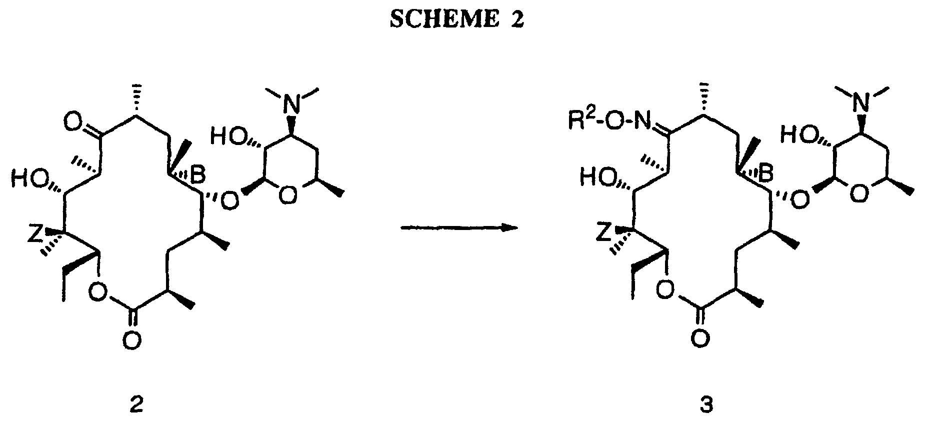

In accordance with Scheme 2 the compound (2) is reacted at its ketone group with an excess of hydroxylamine HCl in the presence of pyridine, or in an alcoholic solution in the presence of a base, such as an alkali metal base, pyridine or a trialkylamine, such as triethylamine, for example, at a temperature of from 50-80°C for 1-4 days, to give compound (3) wherein R² is hydrogen. In the event that compound (3) is desired to be a substituted oxime wherein R² represents C₁-C₆-alkyl, C₁-C₆-alkylamino, aryl-C₁-C₆-alkyl, or heteroaryl-C₁-C₆-alkyl, then compound (2) is reacted with an appropriately substituted hydroxylamine under the conditions described immediately above. Alternately, the compound (3) where R² is hydrogen may be reacted with an appropriate electrophile, such as an alkyl halide, for example, in an aprotic solvent in the presence of an appropriate base, to afford the substituted oxime (3), wherein R² is as described above.

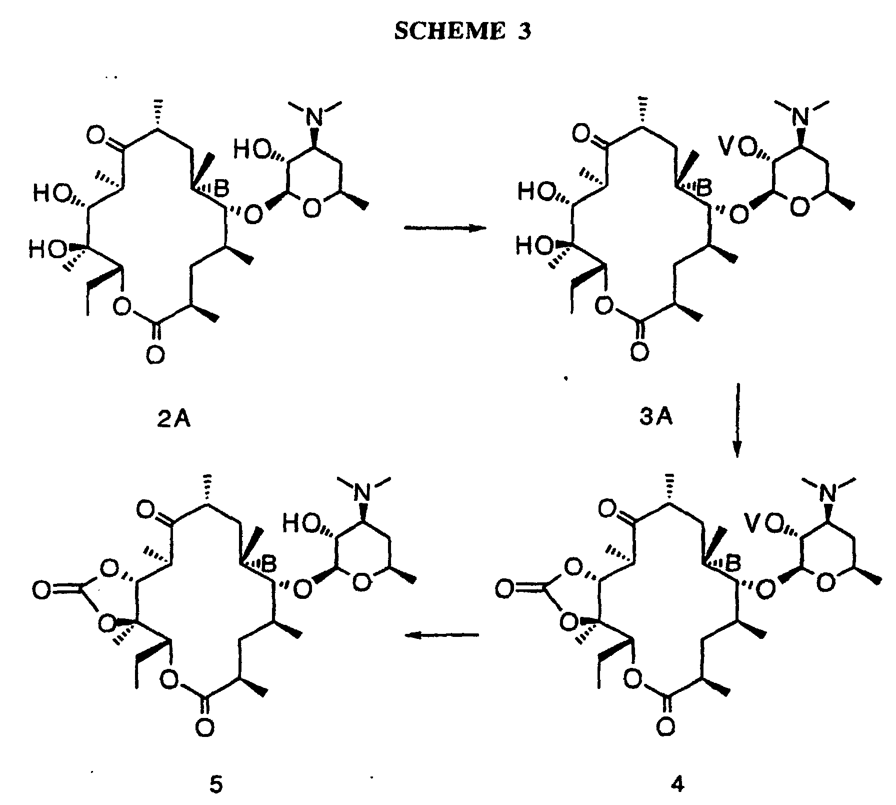

In accordance with Scheme 3, compounds of formula (2A) (*i.e.*, of formula (2) wherein **Z** is hydroxyl) may be reacted in a stepwise manner to give the cyclic carbonate compound (5). First, compound (2A) is reacted with a hydroxy-reactive reagent, such as acetic anydride or benzoic anhydride in the presence of TEA or another base in a solvent such as methylene chloride at ambient or reflux temperatures for 8 to 48 hours, to prepare the intermediate 2'-protected macrolide (3A). Secondly, the 2'-protected intermediate is reacted with a suitable base, such as NaN(TMS)₂, in THF at -40°C, followed by CDI in DMF at room temperature to prepare the 2'-protected-11,12-cyclic carbonate intermediate (4). Lastly, the 2'-protecting group is removed by stirring with methanol for 8-48 hours at ambient to reflux temperature to give the desired compound (5).

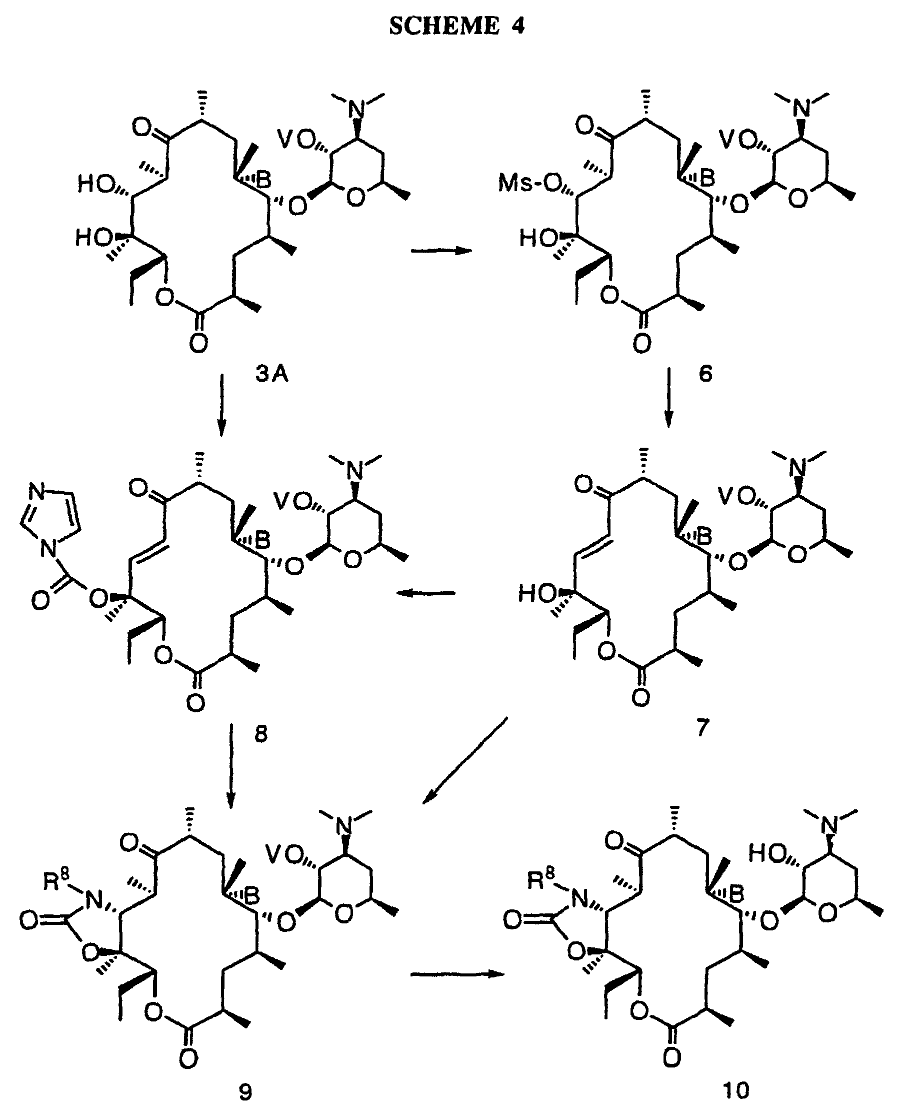

In accordance with Scheme 4 are prepared cyclic carbamate compounds of formula (10), *i.e.,* compounds of Formula (III). First, the 2'-protected compound (3) is reacted with methanesulfonyl anhydride in pyridine at ambient temperature for 4-24 hours to prepare the 2'-protected-11-mesyl intermediate (6). Compound (6) is reacted with a base such as DBU in acetone at ambient temperature for 4-24 hours to give a 2'-protected-10,11-anhydro intermediate (7). Next the 2'-protected-10,11-anhydro intermediate is reacted with a base such as NaN(TMS)₂ in THF at -40°C, followed by CDI in DMF at room temperature to prepare a 12-acylimidazolide-10,11-anhydro intermediate (8). Compound (8) is reacted with an amine in a suitable solvent such as DMF or aqueous acetonitrile to give the 2'-protected compound (9). Lastly, the 2'-protecting group is removed by stirring with methanol for 8-48 hours at ambient temperature to give the desired compound (10). Alternately, compound (8) may be prepared directly from compound (3A) by a base such as NaN(TMS)₂ in THF at -40°C, followed by reaction with an excess of CDI. Also alternately, compound (7) can be directly converted to compound (9) by reaction with an alkyl, aryl, or alkaryl isocyanate, in some cases in the presence of a suitable catalyst such as CuBr, to yield directly the cyclic carbamate (9), wherein Y and Z are as defined above and R⁸ is as defined above but additionally includes aryl and heteroaryl.

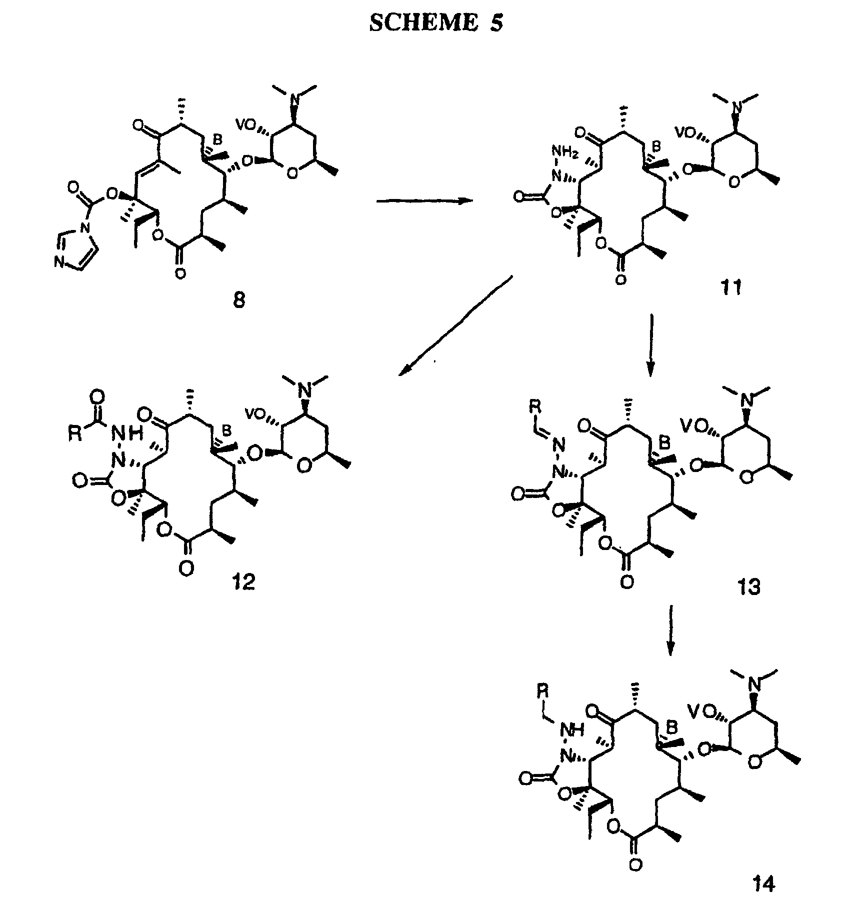

In accordance with Scheme 5 are prepared compounds of Formula (III) wherein R⁸ is NH-R⁹, wherein R⁹ is as defined above. Compound 8 (prepared in Scheme 4) is reacted with an excess of hydrazine in an aprotic solvent, such as DMF or THF, at a temperature from ambient to reflux, for 4 to 24 hours, preferably in DMF at 60°C for 6-10 hours to give compound (11). Compound (11) may then be treated with an acylating reagent, such as an acyl chloride or acyl anhydride, in an aprotic solvent or a carboxylic acid in the presence of EDCI-HCl, triethylamine and DMAP, for example, at ambient to reflux temperatures, for a period of 4-24 hours to give the acylated compound (12). Alternately, compound (11) may be treated with an aldehyde in an aprotic solvent, such as toluene, for example at reflux conditions to prepare the imine compound (13). The imine (13) may then be reduced with sodium cyanoborohydride, for example, in acetic acid and methanol for 6-24 hours to prepare the alkyl compound (14). Alternatively, the imine (13) may be reduced with hydrogen in the presence of a suitable catalyst, using H₂/10% Pd-C for example, in EtOAc or ethanol for 4-48 hours to prepare the alkyl compound (14).

### EXAMPLES

The procedures described above for preparing the compounds of the present invention will be better understood in connection with the following examples, which are intended as an illustration of and not a limitation upon the scope of the invention.

The NMR data for the central portion of the erythromycin compounds exemplified below are given in Table 1, which is placed after Example 8.

### Example 1

### Compound of Formula (I): A=H; B=methoxy; V=H; W and X taken together are =O; Y=Z= -OH

### 1a. 5-O-desosaminyl-6-O-methylerythronolide A

A sample of clarithromycin (3-O-cladinosyl-5-O-desosaminyl-6-O-methylerythronolide A, Abbott Labs, 142.38 g, 190.35 mmol) was suspended in an ethanol-water (1700/600 mL) solution, and 341 mL of 1N HCl was added. The reaction mixture was stirred for 24 hours, and to this solution was then added 170 mL of 2 M NaOH and an additional 250 mL of water with vigorous stirring. The precipitate was collected by filtration, washed with water and dried to afford the title compound (95.00 g, 84%). MS m/z: 590 (M+H)⁺.

### 1b. Compound of Formula (I): A=O-(C=S)-S-CH₃; B=methoxy; V=H; W and X taken together are =O; Y=Z= -OH

To a solution of 5-O-desosaminyl-6-O-methylerythronolide A (11.79 g, 20 mmoL, from step 1a above) in THF (100 mL) at -20°C under an inert atmosphere was added NaH (1.80 g, 60 mmoL, 60% dispersion) slowly over a 5 minute period, and after several minutes CS₂ (1.2 mL, 20 mmoL) was added. After 5 minutes of additional stirring CH₃I (1.24 mL, 20 mmol) was added, and the reaction mixture was allowed to gradually warm to -5 - 0°C. After 1 hour the reaction mixture was diluted with EtOAc (400 mL), then washed with 100 mL portions of saturated aqueous NaHCO₃ (1x) and brine (1x), dried (MgSO₄), and concentrated to afford the crude product as a yellowish foam. Chromatographic purification (silica, CHCl₃; CHCl₃-MeOH (95:5) followed by addition of hexane, filtration, and concentration, afforded the title compound as a white foam (7.68 g; 56%). MS m/z: 680 (M+H)⁺. Anal. Calc'd. for C₃₂H₅₇NO₁₀S₂: C, 56.52; H, 8.45; N, 2.06; Found: C, 56.95; H, 8.65; N, 1.92.

### 1c. Compound of Formula (I): A=H; B=methoxy; V=H: W and X taken together are =O; Y=Z= -OH

A solution of the compound from step 1b (20.00 g; 29.41 mmoL), Bu₃SnH (9.49 mL, 35.29 mmol) and AIBN (∼50 mg, catalytic) in benzene (200 mL) was refluxed (adding 25 mg portions of AIBN periodically) for 8 hours, then the organic layer washed with 100-mL portions of 10% aq KF (1x) and brine (1x), dried (MgSO₄), and concentrated to afford the crude product as an oil. Chromatographic purification (silica, CHCl₃; CHCl₃-MeOH (97.5:2.5) followed by recrystallization from hexane afforded the title compound as a white crystalline material (5.48 g; 32%). MS m/z: 574 (M+H)⁺. Anal. Calc'd. for C₃₀H₅₅NO₉: C, 62.80; H, 9.66; N, 2.44; Found: C, 63.02; H, 9.74; N, 2.30.

### Example 2

### Compound of Formula (I): A=H; B=methoxy; V=H; W and X taken together are =N-OH; Y=Z= -OH;

A sample of the compound of Example 1 (150 mg, 0.26 mmol) and hydroxylamine HCl (72 mg, 1.03 mL, Aldrich) were dissolved in 2 mL of pyridine, and the mixture was heated at 70°C for 24 hours. Additional hydroxylamine HCl (72 mg) was added, and the heating was continued as before for another 24 hours. The solvent was removed under vacuum, and the residue was dissolved in methylene chloride. This solution was washed with saturated aqueous NaHCO₃ solution and brine, dried over MgSO₄ and concentrated to give 112 mg of crude product The residue was purified by flash chromatography on silica gel, eluting with 3-10% methanol in chloroform to afford the title compound (19 mg). MS m/z: 589 (M+H)⁺. High resolution MS; calcd. for C₃₀H₅₇N₂O₉: 589.4064; Found: 589.4046. ¹³C NMR and 1H NMR data are given in Table I below.

### Example 3

### Compound of Formula (II): A=H; B=methoxy; V=H; W and X taken together are =O

### 3a. Compound of Formula (I): A=H; B=methoxy; V=acetyl; W and X taken together are =O; Y=Z= -OH;

A sample of the compound of Example 1 (573 mg, 1.0 mmol), 0.188 mL of acetic anhydride and 0.278 mL of TEA were dissolved in 10 mL of methylene chloride, and the reaction mixture was stirred at room temperature for 20 hours. The reaction mixture was diluted with 40 mL of methylene chloride, and the organic solution was washed with satd. aqueous NaHCO₃ and brine, then dried and concentrated to obtain the title compound (600 mg). MS m/z: 616 (M+H)⁺.

### 3b. Compound of Formula (II): A=H; B=methoxy; V=acetyl; W and X taken together are =O

Under Argon a 150 mg (0.243 mmol) sample of the compound from step 3a was dissolved in THF and cooled to -35°C. NaN(TMS)₂ (0.268 mL, 0.268 mmol, Aldrich, 1.0 M in THF) was added, the reaction mixture was stirred for ten minutes, and a solution of CDI (142 mg, 0.877 mmol, Aldrich) in 2.5 mL of THF was added. The cooling bath was removed, and 15 minutes later the reaction was quenched by the addition of 35 mL of ethyl acetate and 15 mL of satd. aqueous NaHCO₃. The organic layer was separated, washed with brine, dried and concentrated to yield the tide compound (174 mg). MS m/z: 642 (M+H)⁺.

### 3c. Compound of Formula (II): A=H; B=methoxy; V=H; W and X taken together are =O

A 164 mg sample of the compound from step 3b was dissolved in methanol and stirred for 19 hours at room temperature. The solvent was removed under vacuum, and the residue was flash chromatographed on silica gel, eluting with 2.5% methanol in chloroform to afford the title compound (54 mg). MS m/z: 589 (M+H)⁺. Anal. Calc'd. for C₃₁H₅₃NO₁₀: C, 62.08; H, 8.91; N, 2.33; Found: C, 61.80; H, 9.14; N, 2.20.

### Example 4

### Compound of Formula (III): A=H; B=methoxy; V=H; W and X taken together are =O; R¹⁰ = -CH₂CH₂-CH₂-CH₂-phenyl

### 4a. Compound (8) from Scheme 4: B=methoxy; V=acetyl

A sample of the compound from Example 3a above (0.63 g, 1.023 mmol) was dissolved in 10 mL of THF, and the solution was cooled to -60°C. To this stirred solution was added 1.22 mL of sodium bis(trimethyldisilyl) amide (1.0 M in THF). After 4 hours 1,1-carbonyldiimidazole (0.66 g, 4.09 mmol) was added as a solution in 6 mL of 2:3 DMF/THF, and the reaction mixture was slowly warmed to room temperature and stirred for 16 hours. The reaction was quenched by addition of 5% aqueous NaH₂PO₄, and the resulting mixture was extracted with chloroform. The organic layer was washed with water, dried over MgSO₄ and concentrated to give the title compound. MS m/z: 692 (M+H)⁺.

### 4b. Compound of Formula (III): A=H; B=methoxy;V=acetyl; W and X taken together are =O; R¹⁰ = -CH₂-CH₂-CH₂-CH₂-phenyl

A sample of the compound from step 4a (0.1 g, 0.144 mmol) was dissolved in 10% aqueous acetonitrile, 4-phenylbutylamine (0.11 mL, 0.722 mmol) was added, and the reaction mixture was stirred for 16 hours. The solution was diluted with methylene chloride, and the organic layer was separated and washed (3x) with 5% aqueous NaH₂PO₄. The organic layer was dried ever Na₂SO₄ and concentrated, and the residue was purified by flash chromatography on silica gel, eluting with 2-5% ethanol in chloroform to afford 65 mg (59%) of the title compound. MS m/z: 773 (M+H)⁺.

### 4c. Compound of Formula (III): A=H; B=methoxy; V=H; W and X taken together are =O; R¹⁰ = -CH₂-CH₂-CH₂-CH₂-phenyl

A 65 mg sample of the compound from step 4b was dissolved in methanol and stirred at room temperature for 16 hours. The title compound was obtained (45 mg) after filtration and removal of the solvent. MS m/z: 731 (M+H)⁺. High resolution MS: calculated for C₄₁H₆₆N₂O₉: 730.985; found, 730.772. Anal. Calc'd. for C₄₁H₆₆N₂O₉: C, 67.36; H, 9.10; N, 3.83; Found: C, 67.53; H, 9.17; N, 3.94.

### Example 5

### Compound of Formula (III): A=H; B=methoxy; V=H; W and X taken together are =O; R10 = -CH₂-CH₂-phenyl-4-O-phenyl

### 5a. Compound of Formula (III): A=H; B=methoxy; V=acetyl; W and X taken together are =O; R¹⁰ = -CH₂-CH₂-phenyl-4-O-phenyl

A sample of the compound from step 4a (0.18 g, 0.26 mmol) was dissolved in 10% aqueous acetonitrile, 4-phenoxyphenethylamine (0.28 g, 1.3 mmol, Trans World Chemical) was added, and the reaction mixture was stirred for 16 hours. The solution was diluted with methylene chloride, and the mixture was washed (3x) with saturated aqueous NaH₂PO₄. The organic layer was dried over Na₂SO₄ and concentrated, and the residue was purified by flash chromatography on silica gel, eluting with 1-5% ethanol in chloroform to yield 80 mg (36%) of the title compound. MS m/z: 837 (M+H)⁺.

### 5b. Compound of Formula (III): A=H; B=methoxy; V=H; W and X taken together are =O; R¹⁰ = -CH₂-CH₂-phenyl-4-O-phenyl

A 50 mg sample of the compound from step 5a was dissolved in methanol and stirred at room temperature for 16 hours. The desired compound was obtained (40 mg) after filtration and removal of the solvent. MS m/z: 795 (M+H)⁺. Anal. Calc'd. for C₄₅H₆₆N₂O₁₀: C, 67.98; H, 8.36; N, 3.52; Found: C, 67.74; H, 8.46; N, 3.59. The R⁸ ¹³C-NMR peaks are: 157.6, 45.3, 32.1,157.2, 155.3, 133.8,130.1, 129.5, 122.8, 119.0, 118.5, and the ¹H-NMR peaks are: 3.85, 3.79, 2.98, 2.90, 7.49 to 6.79.

### Example 6

### Compound of Formula (I): A=H; B=methoxy; V=H; W and X taken together are =N-O-CH₃; Y=Z= -OH

A sample of the compound from Example 1 (100 mg, 0.174 mmol), methoxylamine HCl (140 mg, 1.74 mmol) and CaCO₃ (110 mg, 1.04 mmol) were suspended in 5 mL of methanol, and the reaction mixture was refluxed under an inert atmosphere for 16 hours. The solvent was removed under vacuum, and the residue was triturated with ethyl acetate. The solvent was filtered, washed with saturated NaHCO₃ and brine, dried over Na₂SO₄ and concentrated. The residue was purified by flash chromatography on silica gel, eluting with chloroform containing 0.5% TEA to give the title compound. MS m/z: 603 (M+H)⁺. The R^{8 13}C-NMR peaks are: 157.6, 43.4, 27.1, 28.6, 35.6, 128.4 to 125.6, and the ¹H-NMR peaks are: : 3.67, 3.59, 1.69, 1.65, 2.67, 7.36-7.04.

### Example 7

### Compound of Formula (I): A=H; B=methoxy; V=H; W and X taken together are =N-O-CH₂-CH₃; Y=Z= -OH

To a sample of the compound from Example 1 (200 mg, 0.348 mmol) dissolved in 2.5 mL of methanol were added ethoxylamine HCl (340 mg, 3.48 mmol) and TEA (0.3 mL, 2.09 mmol), and the reaction mixture was stirred for 16 hours at reflux temperature. The reaction mixture was diluted with chloroform, and the solution was washed with NaHCO₃ and brine, dried over Na₂SO₄ and concentrated. The residue was purified by flash chromatography on silica gel, eluting with 1% methanol in chloroform containing 0.5%-1% TEA to give the title compound. MS m/z: 617 (M+H)⁺. Anal. Calc'd. for C₃₂H₆₀N₂O₉: C, 62.31; H, 9.80; N, 4.54; Found: C, 62.60; H, 9.90; N, 4.89.

### Example 8

### Compound of Formula (I): A=H; B=methoxy; V=H; W and X taken together are =N-O-CH₂-O-CH₂-CH₂-O-CH₃; Y=Z= -OH

A sample of the compound from Example 2 (100 mg, 0.17 mmol), Na₂CO₃ (90 mg, 0.85 mmol) and 2-methoxyethyoxymethyl chloride (0.02 mL, 0.22 mmol) were suspended in 5 mL of acetone and the reaction mixture was stirred for 16 hours at reflux temperature, after which an additional 0.1 mL of 2-methoxyethyoxymethyl chloride was added. The solvent was removed under vacuum, and the residue was triturated with ethyl acetate. The solvent was filtered, washed with saturated aqueous NaHCO₃ and brine, dried over Na₂SO₄ and concentrated. The residue was purified by flash chromatography on silica gel, eluting with chloroform containing 0.5% TEA to give the title compound. MS m/z 677 (M+1).

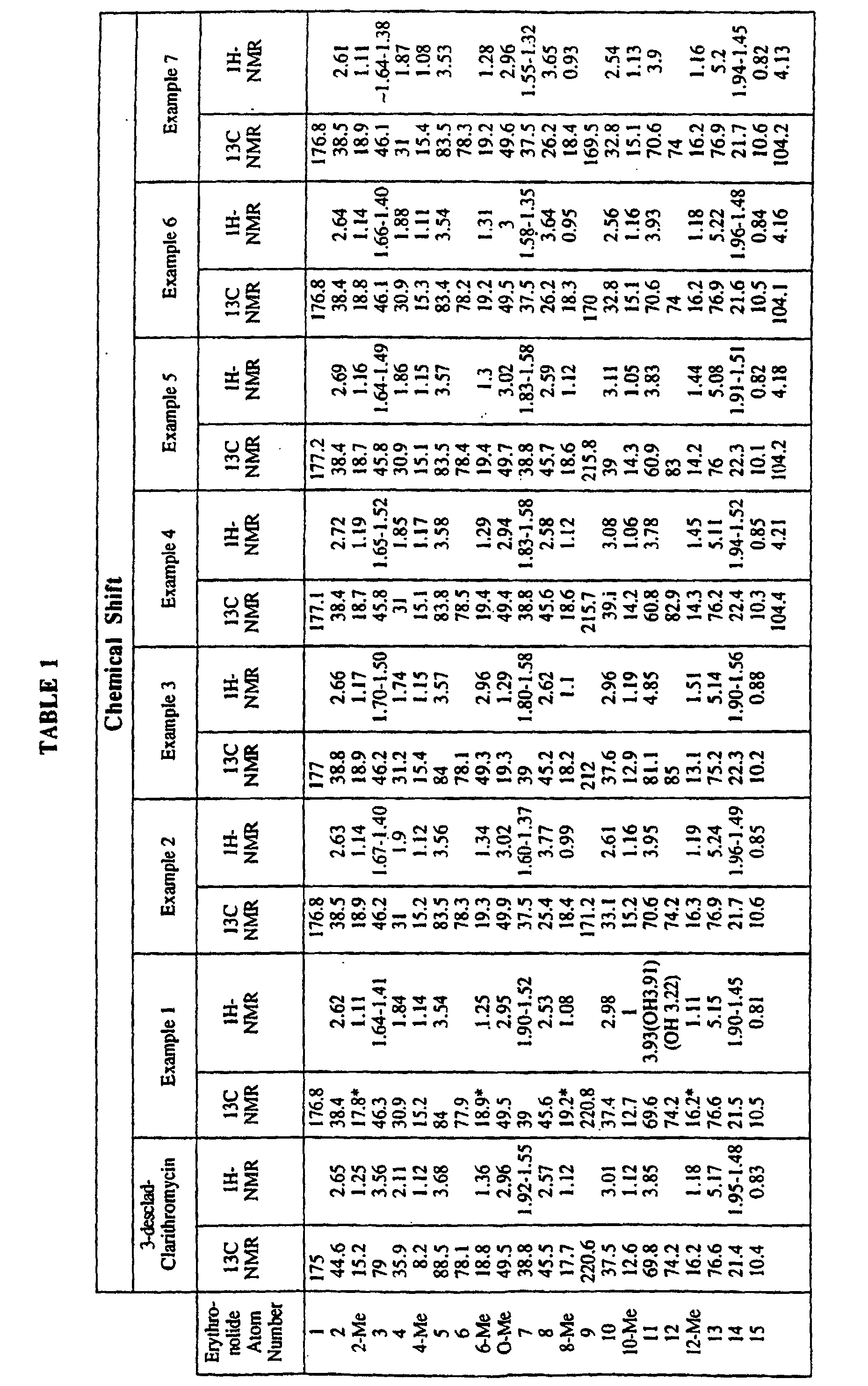

### Example 9

### Compound of Formula (III): A=H; B=methoxy; V=H; W and X taken together are =O; R¹⁰ = NH₂

Hydrazine is added to a solution of the compound from step a of Example 4 in DMF, and the reaction mixture stirred at room temperature. Upon completion of the reaction the mixture is partitioned between EtOAc and H₂O, the layers separated, and the aqueous portion extracted with additional portions of EtOAc. The combined organic layers are then washed with H₂O and brine, dried (Na₂SO₄), and concentrated under reduced pressure. Purification by flash chromatography affords the desired 2'-protected compound. This material is dissolved in MeOH and stirred at room temperature overnight, then the reaction mixture is concentrated under reduced pressure and purified by flash chromatography to give the title compound.

### Example 10

### Compound of Formula (I): A=H; B=hydroxy; V=H; W and X taken together are =O; Y=Z= -OH

### 10a. 5-O-desosaminyl-9-benzyloximo Erythronolide A

A solution of 5-O-desosaminyl erythronolide A oxime (prepared according to the procedure of LeMahieu, *et al., J. Med. Chem.,* 17:953-956, (**1974**)) in THF is treated with NaH followed by benzyl bromide at 0°C under an inert atmosphere, and the reaction mixture is allowed to gradually warm to room temperature. After several hours the reaction mixture is quenched into EtOAc. The organic layer is washed with saturated aqueous NaHCO₃ and brine, dried (MgSO₄), and concentrated to afford the crude product. Chromatographic purification (silica, CHCl₃; CHCl₃-MeOH (95:5) affords the title compound.

### 10b. Compound of Formula (I): A=O-(C=S)-S-CH₃; B=hydroxy; V=H; W and X taken together are =N-O-benzyl; Y=Z= -OH

To a solution of the compound from step 10a in THF at -20°C under an inert atmosphere is added NaH (excess) slowly over a 5 minute period, and after several minutes CS₂ is added. Several minutes later Mel is added, and the reaction mixture allowed to gradually warm to 10°C. After 1 hour the reaction mixture is quenched into EtOAc. The organic layer is washed with saturated aqueous NaHCO₃ and brine, dried (MgSO₄), and concentrated to afford the crude product. Chromatographic purification (silica, CHCl₃; CHCl₃-MeOH affords the title compound.

### 10c. Compound of Formula (I): A=O-(C=S)-S-CH₃; B=hydroxy; V=H; W and X taken together are =N-O-benzyl; Y=Z= -OH

A solution of the compound from step 10b, Bu₃SnH (1.2 eq) and AIBN (catalytic) in benzene is refluxed (adding 25 mg portions of AIBN periodically) for 8 hours, then the organic layer washed with brine (1x), dried (MgSO₄), and concentrated to afford the crude product. Chromatographic purification (silica, CHCl₃; CHCl₃-MeOH) affords the title compound.

### 10d. Compound of Formula (I): A=H; B=hydroxy; V=H; W and X taken together are =O; Y=Z= -OH

A solution of the compound from step 10c in EtOH is hydrogenated using 10% Pd-C under one atmosphere hydrogen, and upon completion of the reaction the mixture is filtered through celite to give the free oxime. De-oximation according to the procedure of LeMahieu, *et al.* (*op. cit.*) followed by chromatographic purification (silica, CHCl₃; CHCl₃-MeOH) affords the title compound.

### Example 11

### Compound of Formula (III): A=H; B=methoxy; V=H; W and X taken together are =O; R⁸ = -NH-CO-CH₃

### 11a. Compound (10) Scheme 4: B=methoxy; V=hydrogen; R⁸=NH₂

Hydrazine is added to a solution of the compound from Example 4 step a in DMF, and the reaction mixture is stirred at room temperature. Upon completion of the reaction the mixture is partitioned between EtOAc and H₂O, the layers are separated, and the aqueous portion is extracted with additional portions of EtOAc. The combined organic layers are then washed with H₂O and brine, dried (Na₂SO₄), and concentrated under reduced pressure. Purification by flash chromatography affords the 2'-acetyl-protected compound. This material is dissolved in MeOH and stirred at room temperature overnight, then the reaction mixture is concentrated under reduced pressure and purified by flash chromatography to give the title compound.

### 11b. Compound of Formula (III): A=H; B=methoxy; V=H; W and X taken together are =O; R⁸ = -NH-CO-CH₃

A sample of the compound from step 11a is dissolved in CH₂Cl₂ and treated with acetic anhydride and 4-dimethylaminopyridine. After stirring under nitrogen for 18 hours, the reaction is quenched by addition of saturated NaHCO₃ solution and extracted into CH₂Cl₂. The organic portion is washed with H₂O and brine, dried (Na₂SO₄), and concentrated under reduced pressure. Purification by flash chromatography gives the 2'-acetyl-protected compound. This material is dissolved in MeOH and stirred at room temperature overnight, then the reaction mixture is concentrated under reduced pressure and purified by flash chromatography to give the title compound.

### Example 12

### Compound of Formula (III): A=H; B=methoxy; V=H; W and X taken together are =O; R⁸ = -N=CH-phenyl

To a sample of the compound from step 11a dissolved in toluene is added benzaldehyde and a small amount of powdered 4Å molecular sieves. The mixture is heated to near reflux under nitrogen for 16 hours, then the reaction mixture is cooled, filtered, and concentrated under reduced pressure. The residue is purified by flash chromatography eluting with MeOH/CH₂Cl₂ to give the tide compound.

### Example 13

### Compound of Formula (III): A=H; B=methoxy; V=H; W and X taken together are =O; R⁸ = -NH₂-CH₂-phenyl

The compound from Example 12 is dissolved in MeOH and 10% Pd on carbon is added. The reaction is then stirred vigorously under 1 atm of H₂ pressure for several days, then the reaction mixture is filtered and concentrated under reduced pressure. Purification of the residue by flash chromatography gives the title compound.

### Example 14

### Compound of Formula (III): A=H; B=methoxy; V=H; W and X taken together are =O; R⁸ = -CH₂-CH₂-CH₂-phenyl

Following the procedure of Example 12, replacing the benzaldehyde thereof with 3-phenylpropanal, the corresponding imine is prepared. This imine compound is dissolved in CH₃CN and reacted with NaCNBH₃ (excess). Acetic acid is added to adjust the pH to 4-6. The reaction is then stirred under nitrogen for 1 day, the reaction is quenched by addition of saturated NaHCO₃ solution, and the product is extracted into CH₂Cl₂. The organic portion is washed with H₂O and brine, dried (Na₂SO₄) and concentrated under reduced pressure. Purification by flash chromatography gives the title compound.

### Examples 15A-15f

Following the procedures of Example 11, except substituting the starting material shown below for the acetic anhydride of Example 11, the title compounds of Examples 15a-15f are prepared as indicated in Table 2 below.

**TABLE 2**

| Ex. No | Starting Material | Title Compound |
|---|---|---|
| 15a | Benzoyl chloride | Compound of Formula III: A=H; B=methoxy; V=H; W and X taken together are O; R⁸ = NH-CO-C₆H₅ |
| 15b | Phenylacetyl chloride | Compound of Formula III: A=H; B=methoxy; V=H; W and X taken together are O; R⁸ = NH-CO-CH₂-C₆H₅ |
| 15c | Phenylpropionyl chloride | Compound of Formula III: A=H; B=methoxy; V=H; W and X taken together are O; R⁸ = NH-CO-CH₂CH₂C₆H₅ |
| 15d | Cinnamoyl chloride | Compound of Formula III: A=H; B=methoxy; V=H; W and X taken together are O; R⁸ = NH-CO-CH₂-CH₂-C₆H₅ |
| 15e | Methylmalonyl chloride | Compound of Formula III: A=H; B=methoxy; V=H; W and X taken together are O; R⁸ = NH-CO-CH₂-COOMe |
| 15f | 3-(4-quinoyl)propionyl chloride | Compound of Formula III: A=H; B=methoxy; V=H; W and X taken together are O; R⁸ = NH-CO-CH₂-CH₂--4-quinoyl) |

### Examples 16a-16xx

Following the procedures of Example 12, except substituting the starting material shown below for the benzaldehyde of Example 12, the title compounds of Examples 16a-16g are prepared as indicated in Table 3 below.

**TABLE 3**

| Ex. No | Starting Material | Title Compound |
|---|---|---|
| 16a | acetaldehyde | Compound of Formula III: A=H; B=methoxy; V=H; W and X taken together are O; R⁸ = N=CH-CH₃ |
| 16b | phenylacetaldehyde | Compound of Formula III: A=H; B=methoxy; V=H; W and X taken together are O; R⁸ = N=CH-CH₂C₆H₅ |
| 16c | phenylpropionaldehyde | Compound of Formula III: A=H; B=methoxy; V=H; W and X taken together are O; R⁸ = N=CH-CH₂CH₂C₆H₅ |
| 16d | cinnamylaldehyde | Compound of Formula III: A=H; B=methoxy; V=H; W and X taken together are O; R⁸ = N=CH-CH=CH-phenyl |
| 16e | 3-(4-quinoyl)propionaldehyde | Compound of Formula III: A=H; B=methoxy; V=H; W and X taken together are O; R⁸ = V=H; W and X taken together are O; R⁸ = N=CH-CH₂-CH₂-(4-quinoyl) |
| 16f | cyclopentanecarboxaldehyde | Compound of Formula III: A=H; B=methoxy; V=H; W and X taken together are O; R⁸ = N=CH-cyclopentane |
| 16f | 2-cyclohexylethanal | Compound of Formula III: A=H; B=methoxy; V=H; W and X taken together are O; R⁸ = N=CH-CH₂-cyclohexane |

### Examples 17a-17

Following the procedures of Example 14, except substituting the starting material shown below for the 3-phenylpropanal of Example 14, the title compounds of Examples 17a-17xx are prepared as indicated in Table 4 below.

**TABLE 4**

| Ex. No | Starting Material | Title Compound |
|---|---|---|
| 17a | acetaldehyde | Compound of Formula III: A=H; B=methoxy; V=H; W and X taken together are O; R⁸ = NH-ethyl |
| 17b | benzaldehyde | Compound of Formula III: A=H; B=methoxy; V=H; W and X taken together are O; R⁸ = NH-CH₂C₆H₅ |
| 17c | phenylacetaldehyde | Compound of Formula III: A=H; B=methoxy; V=H; W and X taken together are O; R⁸ = NH- |
| 17d | phenylpropionaldehyde | Compound of Formula III: A=H; B=methoxy; V=H; W and X taken together are O; R⁸ = NH-CH₂-CH₂-CH₂-C₆H₅ |
| 17e | cinnamylaldehyde | Compound of Formula III: A=H; B=methoxy; V=H; W and X taken together are O; R⁸ = NH-CH₂-CH=CH-phenyl |
| 17f | 3-(4-quinoyl)propionaldehyde | Compound of Formula III: A=H; B=methoxy; V=H; W and X taken together are O; R⁸ = NH-CH₂-CH₂-CH₂-(4-quinoyl) |
| 17g | 2-cyclohexylethanal | Compound of Formula III: A=H; B=methoxy; V=H; W and X taken together are O; R⁸ = NH-CH-CH₂-cyclohexane |

### Examples 18a-18h

Following the procedures of Example 4, except substituting the starting material shown below for the starting material of Example 4, the title compounds of Examples 18a-18h are prepared as indicated in Table 5 below.

**TABLE 5**

| Ex. No | Starting Material | Title Compound |
|---|---|---|
| 18a | ammonia | Compound of Formula III: A=H; B=methoxy; V=H; W and X taken together are O; R⁸ = H |
| 18b | methylamine | Compound of Formula III: A=H; B=methoxy; V=H; W and X taken together are O; R⁸ = Me |
| 18c | benzylamine | Compound of Formula III: A=H; B=methoxy; V=H; W and X taken together are O; R⁸ = benzyl |
| 18d | phenylethylamine | Compound of Formula III: A=H; B=methoxy; V=H; W and X taken together are O; R⁸ = phenylethyl |
| 18e | phenylpropylamine | Compound of Formula III: A=H; B=methoxy; V=H; W and X taken together are O; R⁸ = phenylpropyl |
| 18f | 4-(4-quinoyl)butylamine | Compound of Formula III: A=H; B=methoxy; V=H; W and X taken together are O; R⁸ = -CH₂-CH₂-CH₂-CH₂-(4-quinoyl) |
| 18g | cyclohexylamine | Compound of Formula III: A=H; B=methoxy; V=H; W and X taken together are O; R⁸ = cyclohexyl |
| 18h | 2-cyclohexylethaneamine | Compound of Formula III: A=H; B=methoxy; V=H; W and X taken together are O; R⁸ = -CH₂-CH₂-cyclohexyl |

### Example 19

### Compound of Formula (III): A=H; B=methoxy; V=H; W and X taken together are =O; R⁸ = phenyl

A solution of compound 7 of scheme 4 (B =OMe; V = OAc) in THF is treated with NaH followed by phenylisocyanate and a catalytic amount of Cu(I)Br. After refluxing under nitrogen for 6-24 hours, the reaction is quenched by addition of satd. NaHCO₃ solution and extracted into CH₂Cl₂. The organic portion is washed with H₂O and brine, dried (Na₂SO₄), and concentrated under reduced pressure. Purification by flash chromatography gives the product as the 2'-*O*-acetate. This material is dissolved in MeOH and stirred at r.t. overnight, then the reaction mixture is concentrated under reduced pressure and purified by flash chromatography to give the title compound as a solid.

### Example 19

### In Vitro Assay of Antibacterial Activity

Representative compounds of the present invention were assayed *in vitro* for antibacterial activity as follows: Twelve petri dishes containing successive aqueous dilutions of the test compound mixed with 10 mL of sterilized Brain Heart Infusion (BHI) agar (Difco 0418-01-5) were prepared. Each plate was inoculated with 1:100 (or 1:10 for slow-growing strains, such as *Micrococcus* and *Streptococcus*) dilutions of up to 32 different microorganisms, using a Steers replicator block. The inoculated plates were incubated at 35-37°C for 20 to 24 hours. In addition, a control plate, using BHI agar containing no test compound, was prepared and incubated at the beginning and end of each test.

An additional plate containing a compound having known susceptibility patterns for the organisms being tested and belonging to the same antibiotic class as the test compound was also prepared and incubated as a further control, as well as to provide test-to-test comparability. Erythromycin A was used for this purpose.

After incubation, each plate was examined. The minimum inhibitory concentration (MIC) was defined as the lowest concentration of drug yielding no growth, a slight haze, or sparsely isolated colonies on the inoculum spot as compared to the growth control. The results of this assay, shown below in Table 6 demonstrate the antibacterial activity of the compounds of the invention.

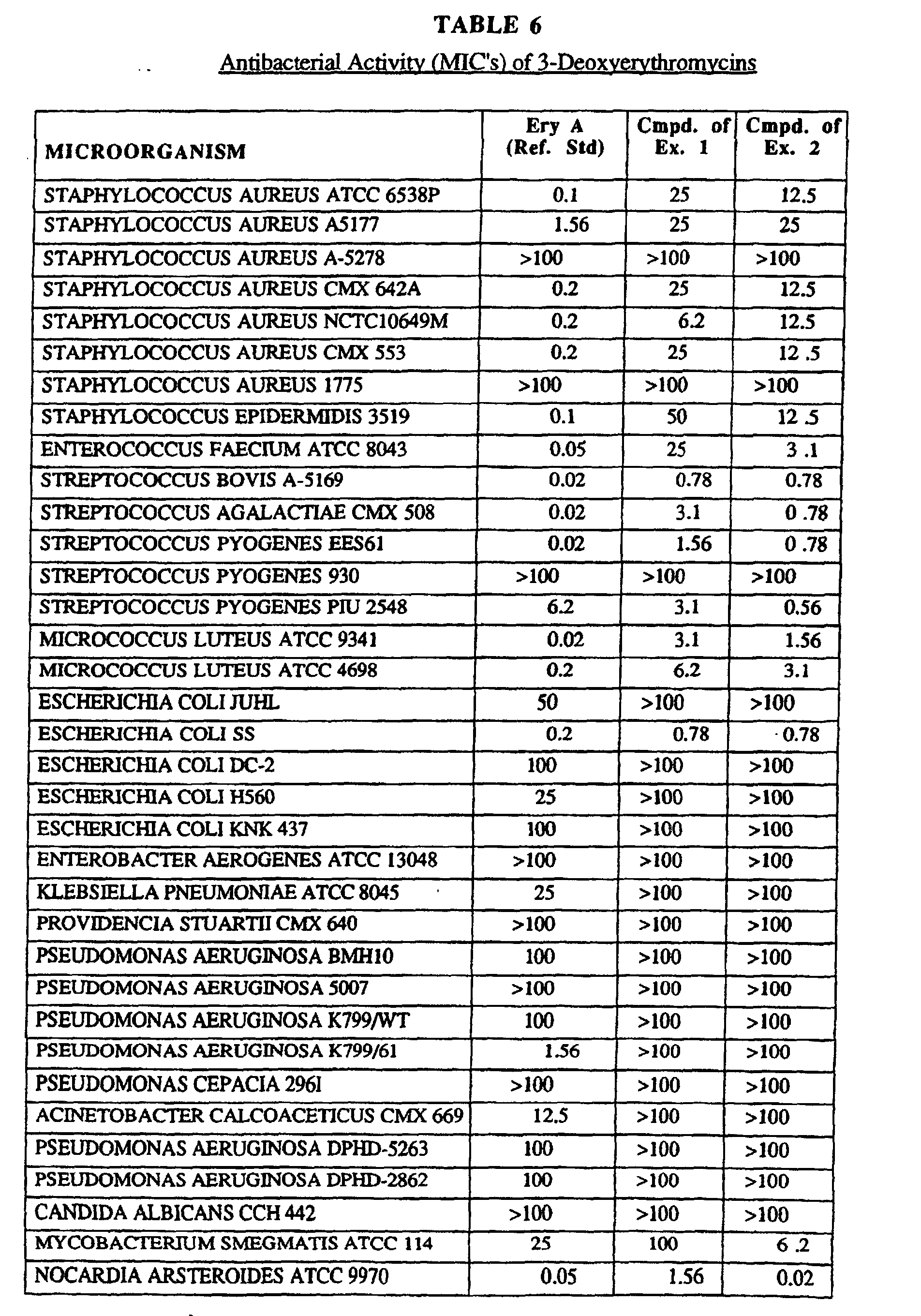

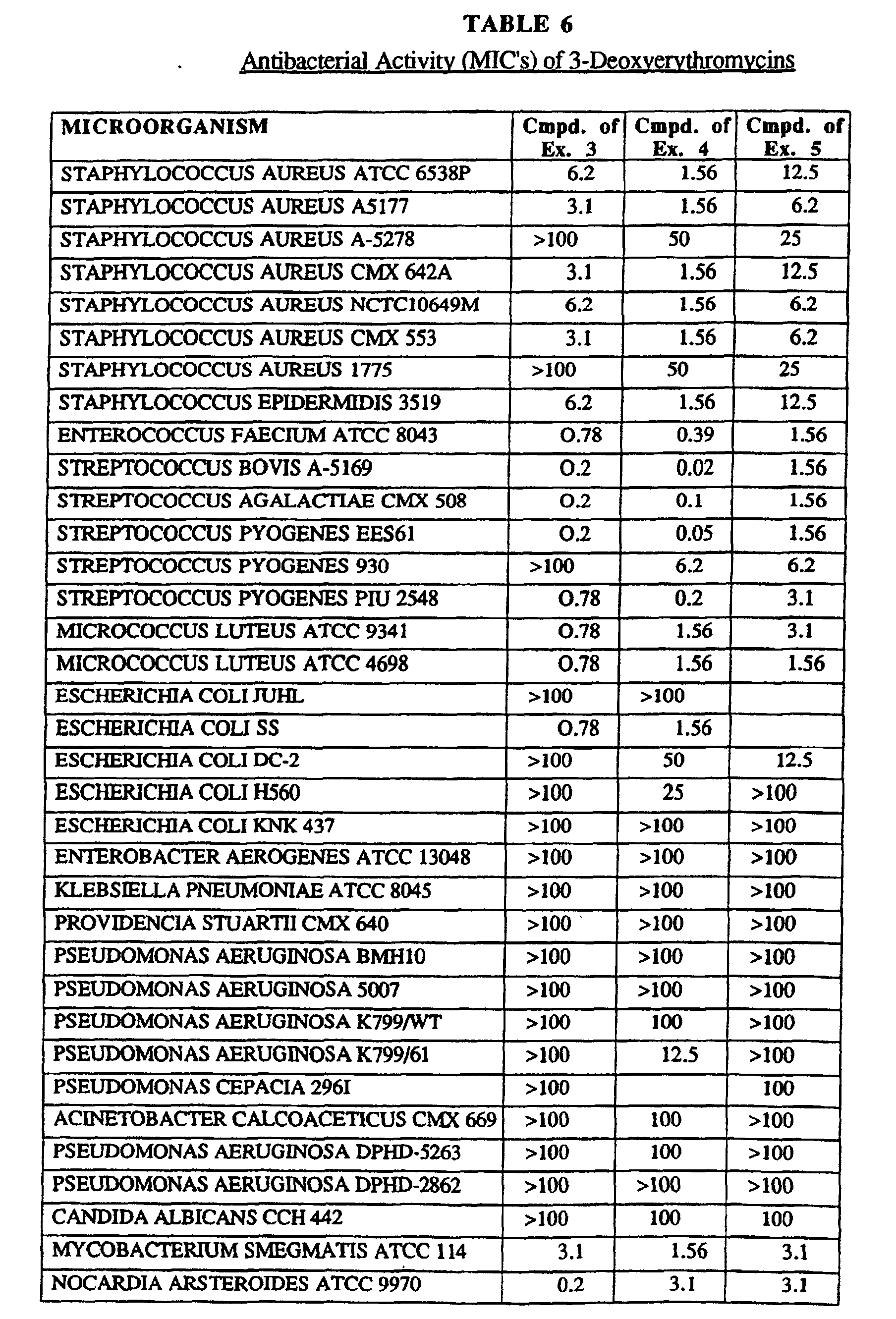

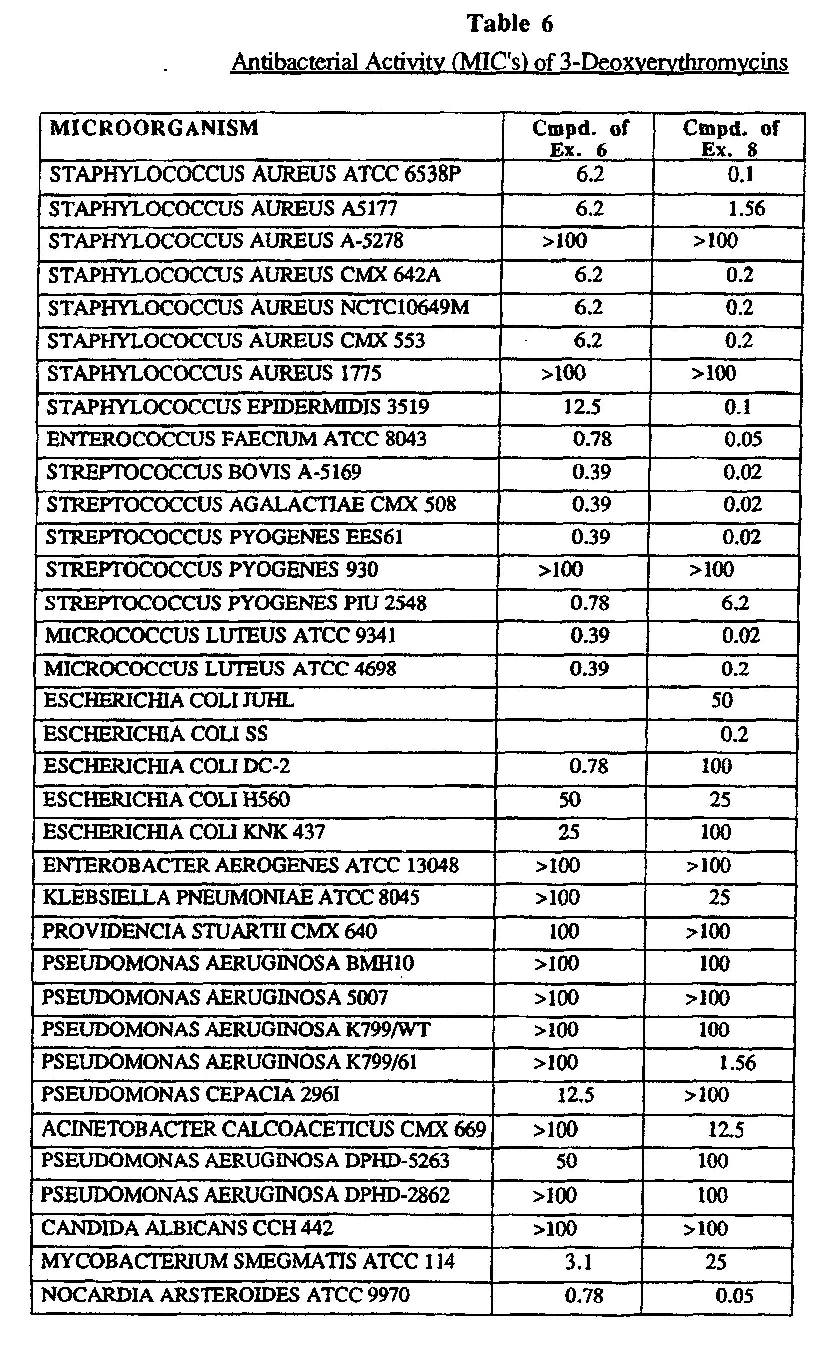

## Claims

1. A compound, or a pharmaceutically acceptable salt or ester thereof, having a formula selected from the group consisting of: 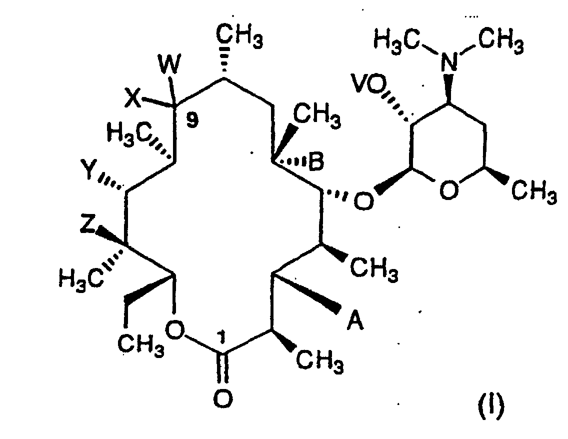 wherein:
**A** is hydrogen,
**B** is hydrogen, hydroxy, or methoxy;
**V** is hydrogen or a hydroxy-protecting group;
**W** is hydrogen; and **X** is selected from the group consisting of:
(a) NR²R³, wherein R² and R³ are independently selected from the group consisting of hydrogen, C₁-C₆-alkyl, C₁-C₆-alkylamino, aryl-C₁-C₆-alkyl or heteroaryl-C₁-C₆-alkyl, or wherein R² and R³ are taken together with the nitrogen atom to which they are attached to form a 5-to-7-membered saturated ring; and
(b) NR4-(C=O)-R¹, wherein R¹ is as described above and R⁴ is hydrogen or C₁-C₆-alkyl; or
**W** and **X** are taken together and represent =O, =N-O-R², wherein R² is as described above, or =N-R⁵, wherein R⁵ is selected from the group consisting of:
(a) hydrogen,
(b) C₁-C₆-alkyl,
(c) substituted C₁-C₆-alkyl,
(d) -(CH₂)ₘ-L-(CH₂)ₙ-M-CH₂-H, wherein m=1-6, n=1-6, L and M are both oxygen, L is oxygen and M is absent, or M is oxygen and L is absent,
(e) -(CH₂)ₘ-L-aryl-M-(CH₂)ₙ-H, wherein m=1-6, n=1-6, L and M are both oxygen, L is oxygen and M is absent, or M is oxygen and L is absent, and
(f) -(CH₂)ₘL-(CH₂)ₙ-M-aryl-H, wherein m=1-6, n=1-6, L and M are both oxygen, L is oxygen and M is absent, or M is oxygen and L is absent;
**Y** is -OH, or -OR⁶ wherein R⁶ is a hydroxy-protecting group; and
**Z** is hydrogen, -OH, or -OR⁷ wherein R⁷ is a hydroxy-protecting group; 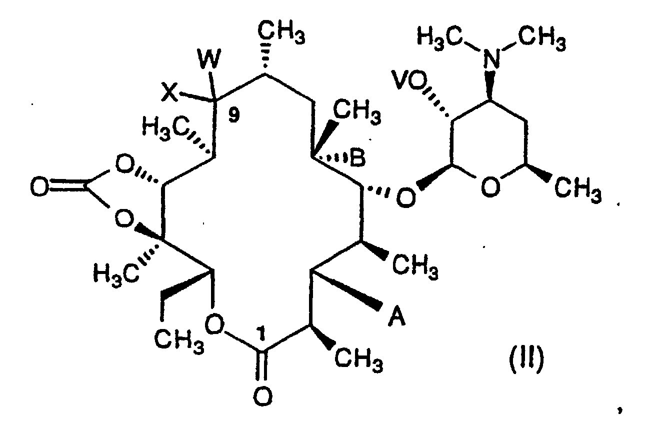
wherein **A, B, V, W,** and **X** are as defined above; and 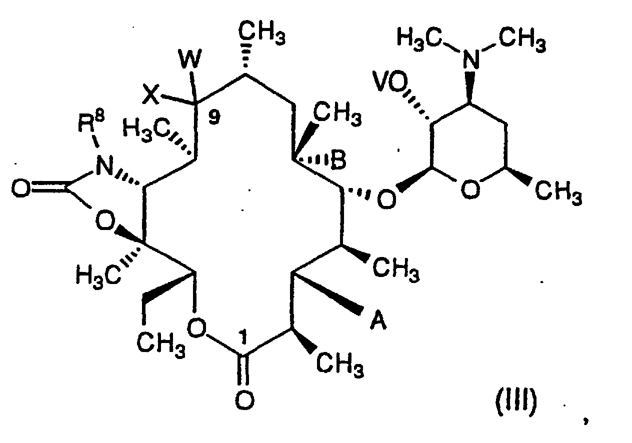 wherein **A, B, V, W,** and **X** are as defined above, and R⁸ is selected from the group consisting of:
(a) hydrogen,
(b) C₁-C₆-alkyl,
(c) aryl,
(d) aryl-C₁-C₆-alkyl,
(e) heteroaryl-C₁-C₆-alkyl,
(f) cycloalkyl,
(g) cycloalkyl-C₁-C₆-alkyl,
(h) -NH-R⁹, wherein R⁹ is selected from the group consisting of:
(aa) C₁-C₆-alkyl,
(bb) aryl-C₁-C₆-alkyl,
(cc) heteroaryl-C₁-C₆-alkyl,
(dd) C₃-C₇-cycloalkyl,
(ee) C₃-C₇-cycloalkyl-C₁-C₆-alkyl,
(ff) -CO-C₁-C₆-alkyl,
(gg) -CO-aryl, and
(hh) -CO-C₁-C₆-alkyl-aryl-; and
(i) -N=CH-R¹⁰, wherein R¹⁰ is selected from the group consisting of:
(aa) C₁-C₆-alkyl,
(bb) aryl,
(cc) aryl-C₁-C₆-alkyl,
(dd) heteroaryl-C₁-C₆-alkyl,
(ee) C₃-C₇-cycloalkyl, and
(ff) C₃-C₇-cycloalkyl-C₁-C₆-alkyl.

2. A compound according to Claim 1 having the formula I.

3. A compound according to Claim 2 wherein A and Z are hydrogen, Y is O-R⁶, wherein R⁶ is hydrogen or a hydroxy-protecting group.

4. A compound according to Claim 2 wherein A is hydrogen and Z is O-R⁷.

5. A compound according to Claim 2 in which:
A=H; B=methoxy; V=H; W and X taken together are =O; Y=Z= -OH;
A=H; B=methoxy; V=H; W and X taken together are =N-OH; Y=Z= -OH;
A=H; B=methoxy; V=H; W and X taken together are =N-O-CH₃; Y=Z= -OH;
A=H; B=methoxy; V=H; W and X taken together are =N-O-CH₂-CH₃; Y=Z= -OH;
A=H; B=methoxy; V=H; W and X taken together are =N-O-CH₂-O-CH₂-CH₂-O-CH₃; Y=Z= -OH; or
A=H; B=hydroxy; V=H; W and X taken together are =O; Y=Z= -OH.

6. A compound according to Claim 1 having the formula (II).

7. A compound according to Claim 6 in which: A=H; B=methoxy; V=H; W and X taken together are =O.

8. A compound according to Claim 1 having the formula (III).

9. A compound according to Claim 8 wherein R⁸ is -NHR⁹, wherein R⁹ is selected from the group consisting of:
(a) C₁-C₆-alkyl;
(b) aryl-C₁-C₆-alkyl;
(c) heteroaryl-C₁-C₆-alkyl;
(d) C₃-C₇-cycloalkyl;
(e) C₃-C₇-cycloalkyl-C₁-C₆-alkyl;
(f) -CO-C₁-C₆-alkyl;
(g) -CO-aryl; and
(h) -CO-C₁-C₆-alkyl-aryl-.

10. A compound according to Claim 9 in which:
A=H; B=methoxy; V=H; W and X taken together are =O; R⁸ = -CH₂-CH₂-CH₂-CH₂-phenyl;
A=H; B=methoxy; V=H; W and X taken together are =O; R⁸ = -CH₂-CH₂-phenyl-4-O-phenyl;
A=H; B=methoxy; V=H; W and X taken together are =O; R⁸ = NH₂;
A=H; B=methoxy; V=H; W and X taken together are =O; R⁸ = -NH-CO-CH₃;
A=H; B=methoxy; V=H; W and X taken together are =O; R⁸ = -N=CH-phenyl;
A=H; B=methoxy; V=H; W and X taken together are =O; R⁸ = -NH₂-CH₂-phenyl;
A=H; B=methoxy; V=H; W and X taken together are =O; R⁸ = -CH₂-CH₂-CH₂-phenyl;
A=H; B=methoxy; V=H; W and X taken together are O; R⁸ = NH-CO-C₆H₅;
A=H; B=methoxy; V=H; W and X taken together are O; R⁸ = NH-CO-CH₂-C₆H₅;
A=H; B=methoxy; V=H; W and X taken together are O; R⁸ = NH-CO-CH₂CH₂C₆H₅;
A=H; B=methoxy; V=H; W and X taken together are O; R⁸ = NH-CO-CH₂--CH₂-C₆H₅;
A=H; B=methoxy; V=H; W and X taken together are O; R⁸ = NH-CO-CH₂-COOMe;
A=H; B=methoxy; V=H; W and X taken together are O; R⁸ = NH-CO-CH₂-CH₂-(4-quinoyl);
A=H; B=methoxy; V=H; W and X taken together are O; R⁸ = N=CH-CH₃;
A=H; B=methoxy; V=H; W and X taken together are O; R⁸ = N=CH-CH₂C₆H₅;
A=H; B=methoxy; V=H; W and X taken together are O; R⁸ = N=CH-CH₂CH₂C₆H₅;
A=H; B=methoxy; V=H; W and X taken together are O; R⁸ = N=CH-CH=CH-phenyl;
A=H; B=methoxy; V=H; W and X taken together are O; R⁸ = N=CH-CH₂-CH₂-(4-quinoyl);
A=H; B=methoxy; V=H; W and X taken together are O; R⁸ = N=CH-cyclopentane;
A=H; B=methoxy; V=H; W and X taken together are O; R⁸ = N=CH-CH₂-cyclohexane;
A=H; B=methoxy; V=H; W and X taken together are O; R⁸ = NH-ethyl;
A=H; B=methoxy; V=H; W and X taken together are O; R⁸ = NH-CH₂C₆H₅;
A=H; B=methoxy; V=H; W and X taken together are O; R⁸ = NH-;
A=H; B=methoxy; V=H; W and X taken together are O; R⁸ = NH-CH₂-CH₂-CH₂-C₆H₅;
A=H; B=methoxy; V=H; W and X taken together are O; R⁸ = NH-CH₂-CH=CH-phenyl;
A=H; B=methoxy; V=H; W and X taken together arc O; R⁸ = NH-CH₂-CH₂-CH₂-(4-quinoyl);
A=H; B=methoxy; V=H; W and X taken together are O; R⁸ = NH-CH-CH₂-cyclohexane;
A=H; B=methoxy; V=H; W and X taken together are O; R⁸ = H;
A=H; B=methoxy; V=H; W and X taken together are O; R⁸ = Me;
A=H; B=methoxy; V=H; W and X taken together are O; R⁸ = benzyl;
A=H; B=methoxy; V=H; W and X taken together are O; R⁸ = phenylethyl;
A=H; B=methoxy; V=H; W and X taken together are O; R⁸ = phenylpropyl;
A=H; B=methoxy; V=H; W and X taken together are O; R⁸ = -CH₂-CH₂-CH₂-CH₂-(4-quinoyl);
A=H; B=methoxy; V=H; W and X taken together are O; R⁸ = cyclohexyl;
A=H; B=methoxy; V=H; W and X taken together are O; R⁸ = -CH₂-CH₂-cyclohexyl; or
A=H; B=methoxy; V=H; W and X taken together are =O; R⁸ = phenyl.

11. A pharmaceutical composition comprising a therapeutically effective amount of compound according to Claim 1 in combination with a pharmaceutically acceptable carrier.

12. Use of a compound according to Claim 1 for preparing a medicament for treating or preventing bacterial infections in a patient in need of such treatment or prevention.

13. A process for the preparation of a compound having the formula: 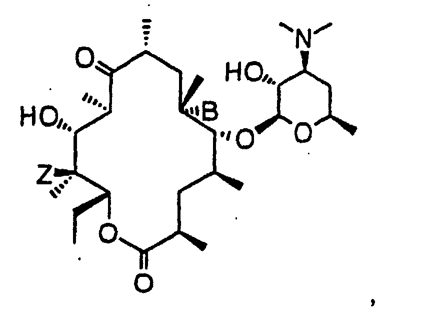 wherein B is hydrogen, hydroxy or methoxy, and Z is H or OH, the method comprising:
(a) reacting a compound having the formula: 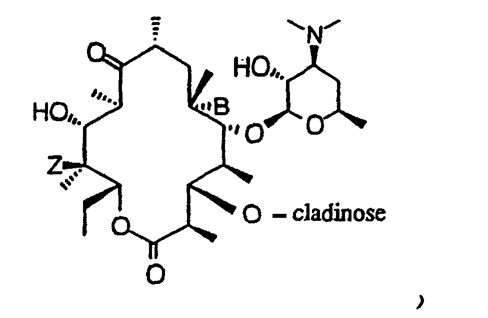 wherein B and Z are as defined above, with a strong acid, preferably 0.5-1.5 N HCl or dichloroacetic acid, in a suspension in aqueous alcohol, preferably, methanol, ethanol, or isopropanol, by stirring at ambient temperature for 0.5 to 24 hours to give a first intermediate compound having the formula: 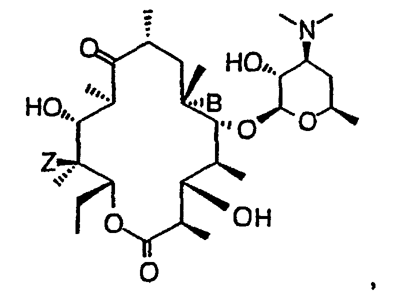 wherein B and Z are as defined above;
(b). reacting the first intermediate compound with an excess of NaH at from 0 to -30°C under an inert atmosphere, in an aprotic solvent such as DMF, THF, or Et₂O, for example, followed by reaction of the intermediate anion with CS₂ and CH₃I at -5 to 10°C, to form a second intermediate 3-O-xanthyl compound having the formula: 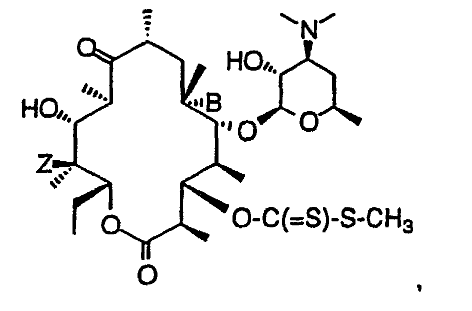 wherein B and Z are as defined above;
(c) reacting the second intermediate compound with 1.1-1.3 equivalents of Bu₃SnH under an inert atmosphere in the presence of a catalytic amount of AIBN or other suitable radical initiator, in a solvent suitable for a free radical reaction, such as benzene or toluene, for example, at reflux conditions, and in the case wherein B = OH and M = NOR², followed by deoximation with, for example, NaNO₂ in the presence of aq. acid/MeOH, or using NaHSO₃/formic acid in H₂O/isopropanol at r.t to reflux to afford the desired compound.

## Revendications

1. Composé, ou sel ou ester pharmaceutiquement acceptable de celui-ci, ayant une formule choisie dans le groupe constitué par : 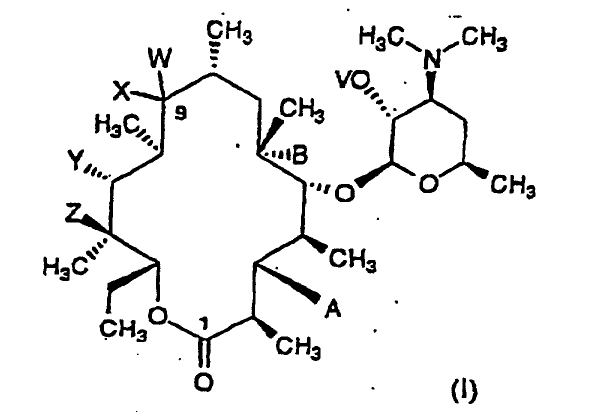 où :
**A** est un hydrogène,
**B** est un hydrogène ou un reste hydroxyle ou méthoxy ;
**V** est un hydrogène ou un groupe protecteur d'hydroxyle ;
**W** est un hydrogène et **X** est choisi dans le groupe constitué par :
(a) NR²R³, où R² et R³ sont choisis, indépendamment l'un de l'autre, dans le groupe constitué par l'hydrogène et les restes alkyle en C₁-C₆, alkylamino en C₁-C₆, aryl(alkyle en C₁-C₆) ou hétéroaryl(alkyle en C₁-C₆), ou bien où R² et R³ considérés ensemble avec l'atome d'azote auquel ils sont attachés forment un cycle saturé à 5 à 7 côtés ; et
(b) NR⁴-(C=O)-R¹, où R¹ est tel que décrit ci-dessus et R⁴ est un hydrogène ou un reste alkyle en C₁-C₆ ; ou
**W** et **X,** considérés ensemble, représentent =O, =N-O-R² où R² est tel que décrit ci-dessus, ou =N-R⁵ où R⁵ est choisi dans le groupe constitué par :
(a) l'hydrogène,
(b) un reste alkyle en C₁-C₆,
(c) un reste alkyle en C₁-C₆ substitué,
(d) -(CH₂)ₘ-L-(CH₂)ₙ-M-CH₂-H, où m = 1 - 6, n = 1 - 6, L et M sont tous deux un oxygène, L est un oxygène et M est absent, ou M est un oxygène et L est absent,
(e) -(CH₂)ₘ-L-aryl-M-(CH₂)ₙ-H, où m = 1 - 6, n = 1 - 6, L et M sont tous deux un oxygène, L est un oxygène et M est absent, ou M est un oxygène et L est absent, et
(f) -(CH₂)ₘ-L-(CH₂)ₙ-M-aryl-H, où m = 1 - 6, n = 1 - 6, L et M sont tous deux un oxygène, L est un oxygène et M est absent, ou M est un oxygène et L est absent ;
**Y** est un reste -OH ou -OR⁶ où R⁶ est un groupe protecteur d'hydroxyle ; et
**Z** est un hydrogène, un reste -OH ou -OR⁷ où R⁷ est un groupe protecteur d'hydroxyle ; 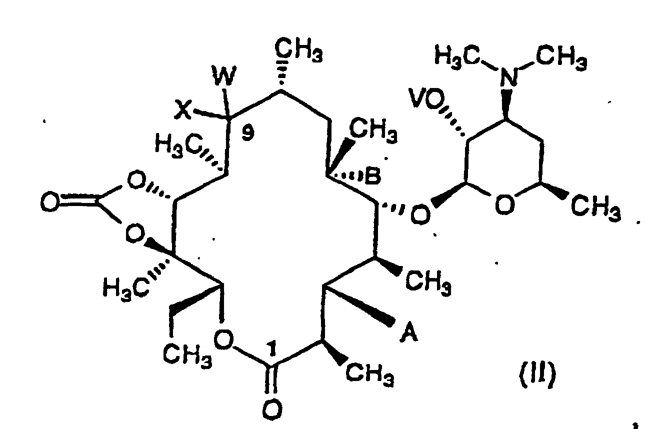
où **A, B, V, W** et **X** sont tels que définis ci-dessus ; et 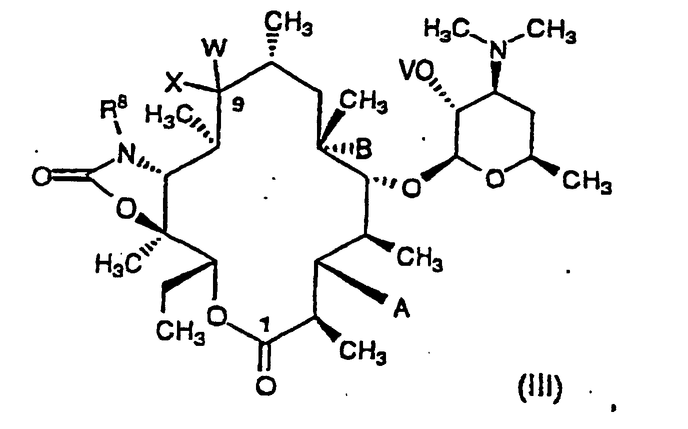 où **A, B, V, W** et **X** sont tels que définis ci-dessus, et R⁸ est choisi dans le groupe constitué par :
(a) l'hydrogène,
(b) un reste alkyle en C₁-C₆,
(c) un reste aryle,
(d) un reste aryl(alkyle en C₁-C₆),
(e) un reste hétéroaryl(alkyle en C₁-C₆),
(f) un reste cycloalkyle,
(g) un reste cycloalkyl(alkyle en C₁-C₆),
(h) -NH-R⁹, où R⁹ est choisi dans le groupe constitué par :
(aa) un reste alkyle en C₁-C₆,
(bb) un reste aryl(alkyle en C₁-C₆),
(cc) un reste hétéroaryl(alkyle en C₁-C₆),
(dd) un reste cycloalkyle en C₃-C₇,
(ee) un reste (cycloalkyl en C₃-C₇)-(alkyle en C₁-C₆),
(ff) un reste -CO-(alkyle en C₁-C₆),
(gg) un reste -CO-aryle et
(hh) un reste -CO-(alkyl en C₁-C₆)-aryle ; et
(i) -N=CH-R¹⁰, où R¹⁰ est choisi dans le groupe constitué par :
(aa) un reste alkyle en C₁-C₆,
(bb) un reste aryle,
(cc) un reste aryl(alkyle en C₁-C₆),
(dd) un reste hétéroaryl(alkyle en C₁-C₆),
(ee) un reste cycloalkyle en C₃-C₇,
(ff) un reste (cycloalkyl en C₃-C₇)-(alkyle en C₁-C₆),

2. Composé selon la revendication 1, ayant la formule (I).

3. Composé selon la revendication 2 où A et Z sont des atomes d'hydrogène, Y est O-R⁶, où R⁶ est un hydrogène ou un groupe protecteur d'hydroxyle.

4. Composé selon la revendication 2 où A est un hydrogène et Z est O-R⁷.

5. Composé selon la revendication 2 dans lequel :
A = H, B = un reste méthoxy ; V = H ; W et X pris ensemble sont =O ; Y = Z = -OH ;
A = H, B = un reste méthoxy ; V = H ; W et X pris ensemble sont =N-OH ; Y = Z = -OH ;
A = H, B = un reste méthoxy ; V = H ; W et X pris ensemble sont =N-O-CH₃ ; Y = Z = -OH ;
A = H, B = un reste méthoxy ; V = H ; W et X pris ensemble sont =N-O-CH₂-CH₃ ; Y = Z = -OH ;
A = H, B = un reste méthoxy ; V = H ; W et X pris ensemble sont =N-O-CH₂-O-CH₂-CH₂-O-CH₃ ; Y = Z = -OH ; ou
A = H, B = un reste hydroxy ; V = H ; W et X pris ensemble sont =O ; Y = Z = -OH ;

6. Composé selon la revendication 1, ayant la formule (II).

7. Composé selon la revendication 6, dans lequel : A = H, B = un reste méthoxy ; V = H ; W et X pris ensemble sont =O.

8. Composé selon la revendication 1, ayant la formule (III).

9. Composé selon la revendication 8, dans lequel R⁸ est -NHR⁹, où R⁹ est choisi dans le groupe constitué par :
(a) un reste alkyle en C₁-C₆,
(b) un reste aryl(alkyle en C₁-C₆),
(c) un reste hétéroaryl(alkyle en C₁-C₆),
(d) un reste cycloalkyle en C₃-C₇,
(e) un reste (cycloalkyl en C₃-C₇)-(alkyle en C₁-C₆),
(f) -CO-(alkyle en C₁-C₆),
(g) -CO-aryle et
(h) -CO-(alkyl en C₁-C₆)-aryle.

10. Composé selon la revendication 9, dans lequel :
A = H, B = un reste méthoxy ; V = H ; W et X pris ensemble sont =O ; R⁸ = -CH₂-CH₂-CH₂-CH₂-phényle ;
A = H, B = un reste méthoxy ; V = H ; W et X pris ensemble sont =O ; R⁸ = -CH₂-CH₂-phényl-4-O-phényle ;
A = H, B = un reste méthoxy ; V = H ; W et X pris ensemble sont =O ; R⁸ = -NH₂ ;
A = H, B = un reste méthoxy ; V = H ; W et X pris ensemble sont =O ; R⁸ = -NH-CO-CH₃ ;
A = H, B = un reste méthoxy ; V = H ; W et X pris ensemble sont =O ; R⁸ = -N=CH-phényle ;
A = H, B = un reste méthoxy ; V = H ; W et X pris ensemble sont =O ; R⁸ = -NH₂-CH₂-phényle ;
A = H, B = un reste méthoxy ; V = H ; W et X pris ensemble sont =O ; R⁸ = -CH₂-CH₂-CH₂-phényle ;
A = H, B = un reste méthoxy ; V = H ; W et X pris ensemble sont =O ; R⁸ = -NH-CO-C₆H₅ ;
A = H, B = un reste méthoxy ; V = H ; W et X pris ensemble sont =O ; R⁸ = -NH-CO-CH₂-C₆H₅ ;
A = H, B = un reste méthoxy ; V = H ; W et X pris ensemble sont =O ; R⁸ = -NH-CO-CH₂CH₂C₆H₅ ;
A = H, B = un reste méthoxy ; V = H ; W et X pris ensemble sont =O ; R⁸ = -NH-CO-CH₂-CH₂-C₆H₅ ;
A = H, B = un reste méthoxy ; V = H ; W et X pris ensemble sont =O ; R⁸ = -NH-CO-CH₂COOMe ;
A = H, B = un reste méthoxy ; V = H ; W et X pris ensemble sont =O ; R⁸ = -NH-CO-CH₂CH₂(4-quinoléyle) ;
A = H, B = un reste méthoxy ; V = H ; W et X pris ensemble sont =O ; R⁸ = -N=CH-CH₃ ;
A = H, B = un reste méthoxy ; V = H ; W et X pris ensemble sont =O ; R⁸ = -N=CH-CH₂C₆H₅ ;
A = H, B = un reste méthoxy ; V = H ; W et X pris ensemble sont =O ; R⁸ = -N=CHCH₂CH₂C₆H₅ ;
A = H, B = un reste méthoxy ; V = H ; W et X pris ensemble sont =O ; R⁸ = -N=CH-CH=CH-phényle ;
A = H, B = un reste méthoxy ; V = H ; W et X pris ensemble sont =O ; R⁸ = -N=CH-CH₂CH₂-(4-quinoléyle) ;
A = H, B = un reste méthoxy ; V = H ; W et X pris ensemble sont =O ; R⁸ = -N=CH-cyclopentane ;
A = H, B = un reste méthoxy ; V = H ; W et X pris ensemble sont =O ; R⁸ = -N=CH-CH₂-cyclohexane ;
A = H, B = un reste méthoxy ; V = H ; W et X pris ensemble sont =O ; R⁸ = -NH-éthyle ;
A = H, B = un reste méthoxy ; V = H ; W et X pris ensemble sont =O ; R⁸ = -NH-CH₂C₆H₅ ;
A = H, B = un reste méthoxy ; V = H ; W et X pris ensemble sont =O ; R⁸ = -NH- ;
A = H, B = un reste méthoxy ; V = H ; W et X pris ensemble sont =O ; R⁸ = -NH-CH₂-CH₂-CH₂-C₆H₅ ;
A = H, B = un reste méthoxy ; V = H ; W et X pris ensemble sont =O ; R⁸ = -NH-CH₂-CH=CH-phényle ;
A = H, B = un reste méthoxy ; V = H ; W et X pris ensemble sont =O ; R⁸ = -NH-CH₂-CH₂-CH₂-(4-quinoléyle) ;
A = H, B = un reste méthoxy ; V = H ; W et X pris ensemble sont =O ; R⁸ = -NH-CH-CH₂-cyclohexane ;
A = H, B = un reste méthoxy ; V = H ; W et X pris ensemble sont =O ; R⁸ = H;
A = H, B = un reste méthoxy ; V = H ; W et X pris ensemble sont =O ; R⁸ = -Me ;
A = H, B = un reste méthoxy ; V = H ; W et X pris ensemble sont =O ; R⁸ = -benzyle ;
A = H, B = un reste méthoxy ; V = H ; W et X pris ensemble sont =O ; R⁸ = -phényléthyle ;
A = H, B = un reste méthoxy ; V = H ; W et X pris ensemble sont =O ; R⁸ = -phénylpropyle ;
A = H, B = un reste méthoxy ; V = H ; W et X pris ensemble sont =O ; R⁸ = -CH₂-CH₂-CH₂-CH₂-(4-quinoléyle) ;
A = H, B = un reste méthoxy ; V = H ; W et X pris ensemble sont =O ; R⁸ = -cyclohexyle ;
A = H, B = un reste méthoxy ; V = H ; W et X pris ensemble sont =O ; R⁸ = -CH₂-CH₂-cyclohexyle ou
A = H, B = un reste méthoxy ; V = H ; W et X pris ensemble sont =O ; R⁸ = -phényle.

11. Composition pharmaceutique comprenant une quantité thérapeutiquement efficace d'un composé selon la revendication 1 en association avec un support pharmaceutiquement acceptable.

12. Utilisation d'un composé selon la revendication 1 pour la préparation d'un médicament pour le traitement ou la prévention d'infections bactériennes chez un patient ayant besoin d'un tel traitement ou d'une telle prévention.

13. Procédé pour la préparation d'un composé ayant pour formule : 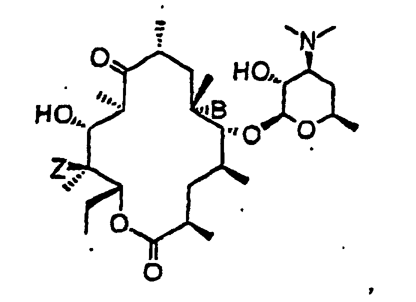 où B est un hydrogène, un reste hydroxy ou méthoxy et Z est H ou OH, la méthode comprenant les étapes consistant à :
(a) faire réagir un composé de formule : 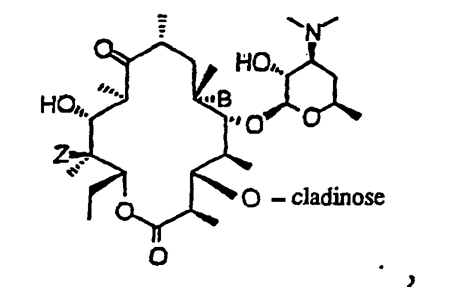 où B et Z sont tels que définis ci-dessus, avec un acide fort, de préférence HCl 0,5 - 1,5 N ou l'acide dichloroacétique, en suspension dans un alcool aqueux, de préférence le méthanol, l'éthanol ou l'isopropanol, sous agitation à la température ambiante pendant 0,5 à 24 heures pour donner un premier composé intermédiaire de formule : 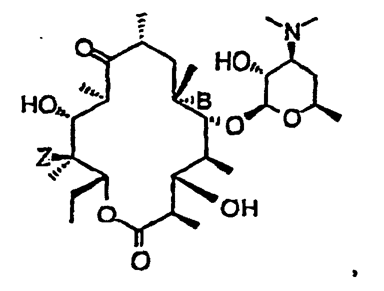 où B et Z sont tels que définis ci-dessus ;
(b) faire réagir le premier composé intermédiaire avec un excès de NaH entre 0 et -30°C sous atmosphère inerte, dans un solvant aprotique tel que le DMF, le THF ou Et₂O, par exemple, puis faire réagir l'anion intermédiaire avec CS₂ et CH₃I entre -5 et 10°C pour former un second composé intermédiaire 3-O-xanthyle de formule : 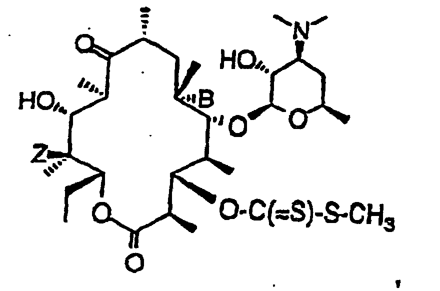 où B et Z sont tels que définis ci-dessus ;
(c) faire réagir le second composé intermédiaire avec 1,1 - 1,3 équivalent de Bu₃SnH sous atmosphère inerte, en présence d'une quantité catalytique d'AlBN ou d'un autre initiateur de radicaux approprié, dans un solvant approprié à une réaction à radicaux libres, comme le benzène ou le toluène, par exemple, dans les conditions du reflux, et dans le cas où B = OH et M = NOR₂, procéder ensuite à une désoximation avec, par exemple, NaNO₂ en présence d'acide aqueux/MeOH ou en utilisant NaHSO₃/acide formique dans H₂O/isopropanol entre la température ambiante et le reflux pour obtenir le composé désiré.

## Patentansprüche

1. Eine Verbindung oder ein pharmazeutisch verträgliches Salz oder Ester davon mit einer Formel, die gewählt ist aus der Gruppe bestehend aus: 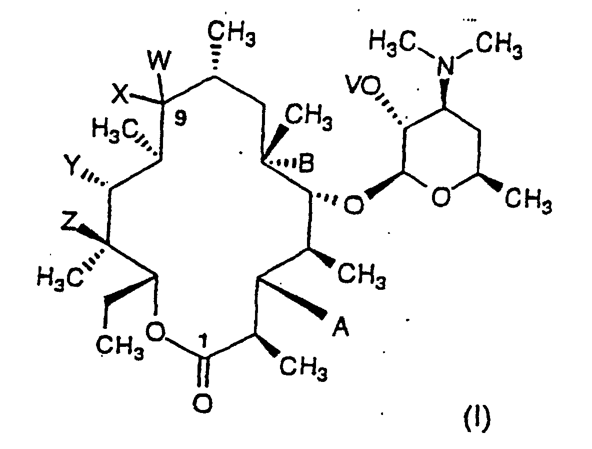 worin
A Wasserstoff ist,
B Wasserstoff, Hydroxy oder Methoxy ist;
V Wasserstoff oder eine Hydroxyschutzgruppe ist;
W Wasserstoff ist; und X ist gewählt aus der Gruppe bestehend aus:
(a) NR²R³, worin R² und R³ unabhängig gewählt sind aus der Gruppe bestehend aus Wasserstoff, C₁-C₆-Alkyl, C₁-C₆-Alkylamino, Aryl-C₁-C₆-Alkyl oder Heteroaryl-C₁-C₆-Alkyl, oder worin R² und R³ zusammengenommen werden mit dem Stickstoffatom, an welches sie angeheftet sind, um einen 5- bis 7-gliedrigen gesättigten Ring zu bilden; und
(b) NR⁴-(C=O)-R¹, worin R¹ wie oben beschrieben ist und R⁴ ist Wasserstoff oder C₁-C₆-Alkyl; oder
W und X werden zusammengenommen und bedeuten =O, =N-O-R², worin R² wie oben beschrieben ist, oder =N-R⁵, worin R⁵ gewählt ist aus der Gruppe bestehend aus:
(a) Wasserstoff,
(b) C₁-C₆-Alkyl,
(c) substituiertes C₁-C₆-Alkyl,
(d) -(CH₂)ₘ-L-(CH₂)ₙ-M-CH₂-H, worin m = 1-6, n = 1-6, L und M beide Sauerstoff sind, L Sauerstoff und M abwesend ist, oder M Sauerstoff und L abwesend ist,
(e) -(CH₂)ₘ-L-Aryl-M-(CH₂)ₙ-H, worin m = 1-6, n = 1-6, L und M beide Sauerstoff sind, L Sauerstoff und M abwesend ist, oder M Sauerstoff und L abwesend ist, und
(f) -(CH₂)ₘ-L-(CH₂)ₙ-M-Aryl-H, worin m = 1-6, n = 1-6, L und M beide Sauerstoff sind, L Sauerstoff und M abwesend ist, oder M Sauerstoff und L abwesend ist;
X ist -OH, oder -OR⁶, worin R⁶ eine Hydroxyschutzgruppe ist; und
Z ist Wasserstoff, -OH, oder -OR⁷, worin R⁷ eine Hydroxyschutzgruppe ist; 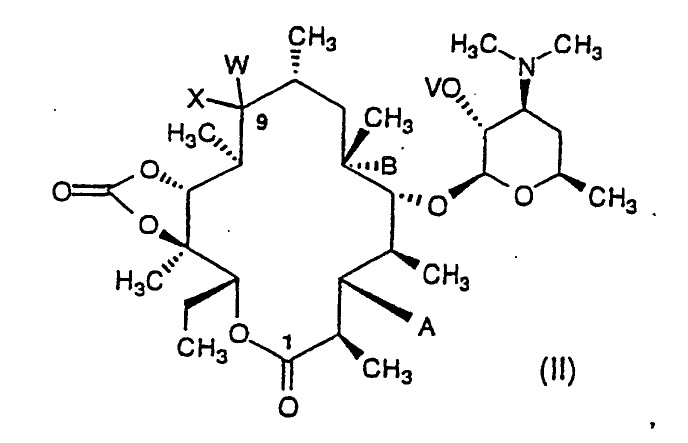
worin A, B, V, W und X wie oben definiert sind; und 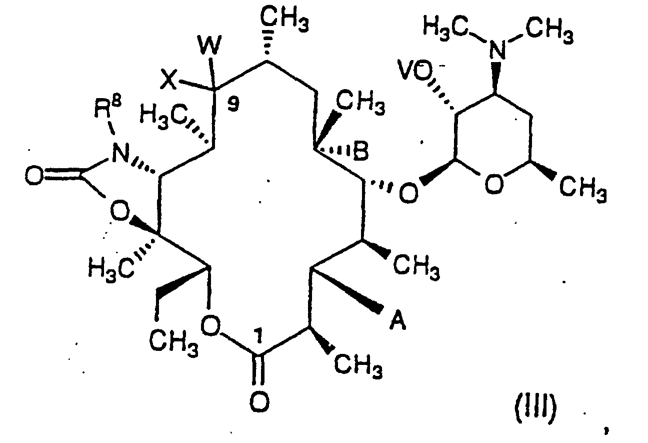 worin A, B, V, W und X wie oben definiert sind, und R⁸ ist gewählt aus der Gruppe bestehend aus:
(a) Wasserstoff,
(b) C₁-C₆-Alkyl,
(c) Aryl,
(d) Aryl-C₁-C₆-Alkyl,
(e) Heteroaryl-C₁-C₆-Alkyl,
(f) Cycloalkyl,
(g) Cycloalkyl-C₁-C₆-Alkyl,
(h) -NH-R⁹, worin R⁹ gewählt ist aus der Gruppe bestehend aus:
(aa) C₁-C₆-Alkyl,
(bb) Aryl-C₁-C₆-Alkyl,
(cc) Heteroaryl-C₁-C₆-Alkyl,
(dd) C₃-C₇-Cycloalkyl,
(ee) C₃-C₇-Cycloalkyl-C₁-C₆-Alkyl,
(ff) -CO-C₁-C₆-Alkyl,
(gg) -CO-Aryl, und
(hh) -CO-C₁-C₆-Alkyl-Aryl-; und
(i) -N=CH-R¹⁰, worin R¹⁰ gewählt ist aus der Gruppe bestehend aus:
(aa) C₁-C₆-Alkyl,
(bb) Aryl,
(cc) Aryl-C₁-C₆-Alkyl,
(dd) Heteroaryl-C₁-C₆-Alkyl,
(ee) C₃-C₇-Cycloalkyl, und
(ff) C₃-C₇-Cycloalkyl-C₁-C₆-Alkyl.

2. Eine Verbindung gemäß Anspruch 1 mit der Formel I.

3. Eine Verbindung gemäß Anspruch 2, worin A und Z Wasserstoff sind, Y O-R⁶ ist, worin R⁶ Wasserstoff oder eine Hydroxyschutzgruppe ist.

4. Eine Verbindung gemäß Anspruch 2, worin A Wasserstoff und Z O-R⁷ ist.

5. Eine Verbindung gemäß Anspruch 2, worin:
A=H; B=Methoxy; V=H; W und X zusammengenommen =O sind; Y=Z= -OH;
A=H; B=Methoxy; V=H; W und X zusammengenommen =N-OH sind; Y=Z= -OH;
A=H; B=Methoxy; V=H; W und X zusammengenommen =N-O-CH₃ sind; Y=Z= -OH;
A=H; B=Methoxy; V=H; W und X zusammengenommen =N-O-CH₂-CH₃ sind; Y=Z= -OH;
A=H; B=Methoxy; V=H; W und X zusammengenommen =N-O-CH₂-O-CH₂-CH₂-O-CH₃ sind; Y=Z= -OH; oder
A=H; B=Hydroxy; V=H; W und X zusammengenommen =O sind; Y=Z= -OH.

6. Eine Verbindung gemäß Anspruch 1 mit der Formel (II).

7. Eine Verbindung gemäß Anspruch 6, worin A=H; B=Methoxy; V=H; W und X zusammengenommen =O sind.

8. Eine Verbindung gemäß Anspruch 1 mit der Formel (III).

9. Eine Verbindung gemäß Anspruch 8, worin R⁸ -NHR⁹ ist, worin R⁹ gewählt ist aus der Gruppe bestehend aus:
(a) C₁-C₆-Alkyl,
(b) Aryl-C₁-C₆-Alkyl,
(c) Heteroaryl-C₁-C₆-Alkyl,
(d) C₃-C₇-Cycloalkyl,
(e) C₃-C₇-Cycloalkyl-C₁-C₆-Alkyl,
(f) -CO-C₁-C₆-Alkyl,
(g) -CO-Aryl, und
(h) -CO-C₁-C₆-Alkyl-Aryl-.

10. Eine Verbindung gemäß Anspruch 9, worin:
A=H; B=Methoxy; V=H; W und X zusammengenommen =O sind; R⁸ = -CH₂-CH₂-CH₂-CH₂-Phenyl;
A=H; B=Methoxy; V=H; W und X zusammengenommen =O sind; R⁸ = -CH₂-CH₂-Phenyl-4-O-Phenyl;
A=H; B=Methoxy; V=H; W und X zusammengenommen =O sind; R⁸ = -NH₂;
A=H; B=Methoxy; V=H; W und X zusammengenommen =O sind; R⁸ = -NH-CO-CH₃;
A=H; B=Methoxy; V=H; W und X zusammengenommen =O sind; R⁸ = -N=CH-Phenyl;
A=H; B=Methoxy; V=H; W und X zusammengenommen =O sind; R⁸ = -NH₂-CH₂-Phenyl;
A=H; B=Methoxy; V=H; W und X zusammengenommen =O sind; R⁸ = -CH₂-CH₂-CH₂-Phenyl;
A=H; B=Methoxy; V=H; W und X zusammengenommen =O sind; R⁸ = NH-CO-C₆H₅;
A=H; B=Methoxy; V=H; W und X zusammengenommen =O sind; R⁸ = NH-CO-CH₂-C₆H₅;
A=H; B=Methoxy; V=H; W und X zusammengenommen =O sind; R⁸ = NH-CO-CH₂CH₂C₆H₅;
A=H; B=Methoxy; V=H; W und X zusammengenommen =O sind; R⁸ = NH-CO-CH₂-CH₂-C₆H₅;
A=H; B=Methoxy; V=H; W und X zusammengenommen =O sind; R⁸ = NH-CO-CH₂-COOMe;
A=H; B=Methoxy; V=H; W und X zusammengenommen =O sind; R⁸ = NH-CO-CH₂-CH₂-(4-Chinoyl);
A=H; B=Methoxy; V=H; W und X zusammengenommen =O sind; R⁸ = N=CH-CH₃;
A=H; B=Methoxy; V=H; W und X zusammengenommen =O sind; R⁸ = N=CH-CH₂C₆H₅;
A=H; B=Methoxy; V=H; W und X zusammengenommen =O sind; R⁸ = N=CH-CH₂CH₂C₆H₅;
A=H; B=Methoxy; V=H; W und X zusammengenommen =O sind; R⁸ = N=CH-CH=CH-Phenyl;
A=H; B=Methoxy; V=H; W und X zusammengenommen =O sind; R⁸ = N=CH-CH₂-CH₂-(4-Chinoyl);
A=H; B=Methoxy; V=H; W und X zusammengenommen =O sind; R⁸ = N=CH-Cyclopentan;
A=H; B=Methoxy; V=H; W und X zusammengenommen =O sind; R⁸ = N=CH-CH₂-Cyclohexan;
A=H; B=Methoxy; V=H; W und X zusammengenommen =O sind; R⁸ = NH-Ethyl;
A=H; B=Methoxy; V=H; W und X zusammengenommen =O sind; R⁸ = NH-CH₂ C₆H₅;
A=H; B=Methoxy; V=H; W und X zusammengenommen =O sind; R⁸ = NH-;
A=H; B=Methoxy; V=H; W und X zusammengenommen =O sind; R⁸ = NH-CH₂-CH₂-CH₂-C₆H₅;
A=H; B=Methoxy; V=H; W und X zusammengenommen =O sind; R⁸ = NH-CH₂-CH=CH-Phenyl;
A=H; B=Methoxy; V=H; W und X zusammengenommen =O sind; R⁸ = NH-CH₂-CH₂-CH₂-(4-Chinoyl);
A=H; B=Methoxy; V=H; W und X zusammengenommen =O sind; R⁸ = NH-CH-CH₂-Cyclohexan;
A=H; B=Methoxy; V=H; W und X zusammengenommen =O sind; R⁸ = H;
A=H; B=Methoxy; V=H; W und X zusammengenommen =O sind; R⁸ = Me;
A=H; B=Methoxy; V=H; W und X zusammengenommen =O sind; R⁸ = Benzyl;
A=H; B=Methoxy; V=H; W und X zusammengenommen =O sind; R⁸ = Phenylethyl;
A=H; B=Methoxy; V=H; W und X zusammengenommen =O sind; R⁸ = Phenylpropyl;
A=H; B=Methoxy; V=H; W und X zusammengenommen =O sind; R⁸ = -CH₂-CH₂-CH₂-CH₂-(4-Chinoyl);
A=H; B=Methoxy; V=H; W und X zusammengenommen =O sind; R⁸ = Cyclohexyl;
A=H; B=Methoxy; V=H; W und X zusammengenommen =O sind; R⁸ = -CH₂-CH₂-Cyclohexyl; oder
A=H; B=Methoxy; V=H; W und X zusammengenommen =O sind; R⁸ = Phenyl;

11. Eine pharmazeutische Zusammensetzung, die eine therapeutisch wirksame Menge einer Verbindung gemäß Anspruch 1 in Kombination mit einem pharmazeutisch verträglichen Träger umfaßt.

12. Verwendung einer Verbindung gemäß Anspruch 1 zur Herstellung eines Medikaments zur Behandlung oder zur Vorbeugung von bakteriellen Infektionen in einem Patienten, der einer solchen Behandlung oder vorbeugenden Maßnahme bedarf.

13. Ein Verfahren zur Herstellung einer Verbindung mit der Formel: 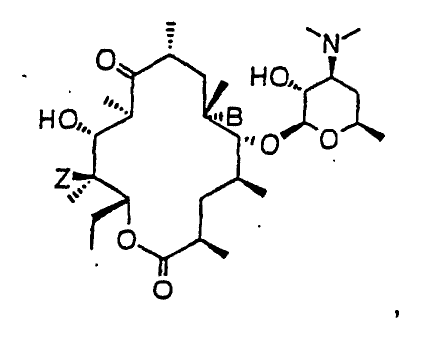 worin B Wasserstoff, Hydroxy oder Methoxy ist, und Z ist H oder OH, wobei das Verfahren folgendes umfaßt:
(a) Reagieren einer Verbindung mit der Formel: 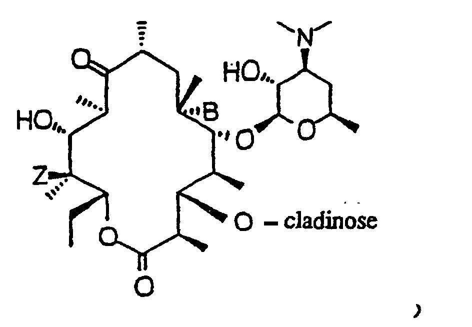 worin B und Z wie oben definiert sind, mit einer starken Säure, vorzugsweise 0,5-1,5 N HCl oder Dichloressigsäure, in einer Suspension in wässerigem Alkohol, vorzugsweise Methanol, Ethanol oder Isopropanol, durch Rühren bei Raumtemperatur für 0,5 bis 24 Stunden, um eine erste intermediäre Verbindung mit folgender Formel zu ergeben: 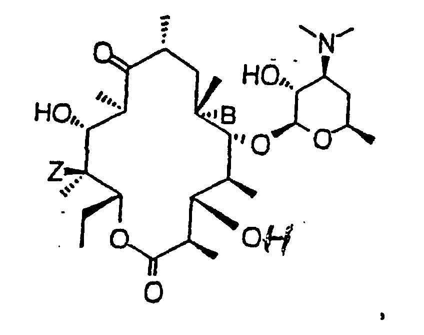 worin B und Z wie oben definiert sind;
(b) Reagieren der ersten intermediären Verbindung mit einem Überschuß an NaH bei von 0 bis -30°C unter einer inerten Atmosphäre, in einem aprotischen Lösungsmittel wie beispielsweise DMF, THF oder ET₂O, z.B. gefolgt von der Reaktion des intermediären Anions mit CS₂ und CH₃I bei -5 bis 10°C, um eine zweite intermediäre 3-O-Xanthyl-Verbindung mit folgender Formel zu bilden: 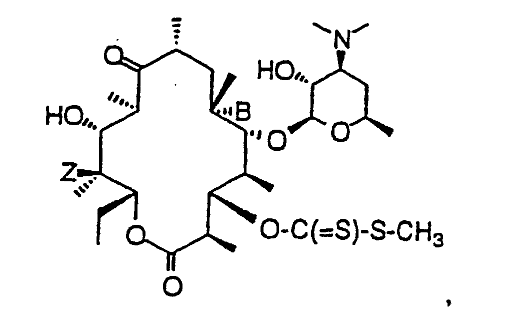 worin B und Z wie oben definiert sind;
(c) Reagieren der zweiten intermediären Verbindung mit 1,1 - 1,3 Äquivalenten von Bu₃SnH unter einer inerten Atmosphäre in der Anwesenheit einer katalytischen Menge an AIBN oder einem anderen geeigneten Radikalinitiator, in einem für eine freie Radikalreaktion geeigneten Lösungsmittel wie beispielsweise Benzen oder Toluen, z.B. unter Rückflussbedingungen, und in dem Fall worin B = OH und M = NOR², gefolgt von Deoximation mit beispielsweise NaNO₂ in Anwesenheit von wässer. Säure/MeOH, oder unter Verwendung von NaHSO₃/Ameisensäure in H₂O/Isopropanol bei Rt unter Rückfluss, um die gewünschte Verbindung zu liefern.
